(19) **Europäisches Patentamt / European Patent Office / Office européen des brevets**

(11) **EP 4 316 656 A1**

(12) # EUROPEAN PATENT APPLICATION
published in accordance with Art. 153(4) EPC

(43) Date of publication:
**07.02.2024 Bulletin 2024/06**

(21) Application number: **21933136.0**

(22) Date of filing: **26.03.2021**

(51) International Patent Classification (IPC):
**B01J 23/89** (2006.01)        **C07C 67/39** (2006.01)
**C07C 69/54** (2006.01)

(52) Cooperative Patent Classification (CPC):
**B01J 23/89; C07C 67/39; C07C 69/54**

(86) International application number:
**PCT/JP2021/013068**

(87) International publication number:
**WO 2022/201533 (29.09.2022 Gazette 2022/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Asahi Kasei Kabushiki Kaisha Tokyo 1000006 (JP)**

(72) Inventors:
- **NAGATA, Dai Tokyo 100-0006 (JP)**
- **IITSUKA, Chihiro Tokyo 100-0006 (JP)**

(74) Representative: **dompatent von Kreisler Selting Werner - Partnerschaft von Patent- und Rechtsanwälten mbB Deichmannhaus am Dom Bahnhofsvorplatz 1 50667 Köln (DE)**

(54) **CATALYST FOR PRODUCTION OF CARBOXYLIC ACID ESTER, METHOD FOR PRODUCING CARBOXYLIC ACID ESTER, AND METHOD FOR PRODUCING CATALYST FOR PRODUCTION OF CARBOXYLIC ACID ESTER**

(57)    A catalyst for production of carboxylic acid ester, containing:
catalyst metal particles; and
a support supporting the catalyst metal particles,
wherein a bulk density of the catalyst for production of carboxylic acid ester is 0.50 g/cm$^3$ or more and 1.50 g/cm$^3$ or less,
when a particle diameter, at which a cumulative frequency is x% in a particle diameter distribution based on a volume of the catalyst for production of carboxylic acid ester, is defined as $D_x$, $D_{10}/D_{50} \geq 0.2$ and $D_{90}/D_{50} \leq 2.5$ are satisfied, and
when a half-width of the particle diameter distribution is defined as W, $W/D_{50} \leq 1.5$ is satisfied.

**Description**

Technical Field

The present invention relates to a catalyst for production of carboxylic acid ester, a method for producing carboxylic acid ester, and a method for producing a catalyst for production of carboxylic acid ester.

Background Art

Nickel or nickel compounds are widely used as catalysts for chemical synthesis such as oxidation reactions, reduction reactions, or hydrogenation reactions. In recent years, catalytic oxygen oxidation reactions of alcohols have been realized by various modifications or improvements of nickel-based catalysts. In this context, such nickel and nickel compounds are known in the chemical industry to be widely effective not only for the oxidation reactions of alcohols but also for various reactions such as various oxidation reactions, reduction reactions, and hydrogenation reactions, and for purification catalysts for vehicle emissions, photocatalysts, and the like.

For example, in Patent Literature 1, as a method for producing carboxylic acid ester, there is suggested use of a composite particle supported material as a catalyst, the composite particle supported material comprising: composite particles constituted by oxidized nickel and X (wherein X represents at least one element selected from the group consisting of nickel, palladium, platinum, ruthenium, gold, silver, and copper); and a support supporting the composite particles, wherein the composite particle supported material has a supported layer in which the composite particles are localized. The catalyst is said to be capable of maintaining high reactivity over a long period.

Patent Literature 2 describes spherical silica particles containing aluminum and magnesium, wherein a specific surface area, a pore volume, a pore distribution, a bulk density, abrasion resistance, an average particle diameter, an aluminum content, and a magnesium content are set to predetermined ranges. According to the literature, it is said that spherical silica-based particles can be provided which may be used for a wide range of purposes including catalyst supports, have a high mechanical strength, have a large specific surface area, and have good fluidity.

Citation List

Patent Literature

Patent Literature 1: Japanese Patent No. 4803767
Patent Literature 2: Japanese Patent No. 4420991

Summary of Invention

Technical Problem

In production of carboxylic acid ester using the catalyst described in Patent Literature 1 or 2, improvement in catalyst activity is demanded, and a catalyst that exhibits a higher conversion rate is demanded. The present inventors have conducted experiments on production of carboxylic acid ester and consequently found that when carboxylic acid ester is produced over a long period using the catalyst described in Patent Literature 1 or 2, the conversion rate of a raw material tends to be gradually decreased. As a result of conducting more detailed studies on a cause thereof, it has been found that outflow of the catalyst from a reactor or fluidity of the catalyst influences the conversion rate of a raw material. The conversion rate of a raw material or the selectivity of carboxylic acid ester can be improved by increasing the amount of air blown at the time of production of carboxylic acid ester. However, if the catalyst easily outflows from a reactor, the influence of catalyst outflow is more prominent with increase in the amount of air blown. In this respect as well, improvement in reaction results may be hindered.

The present invention has been accomplished in light of the above problems involved in the conventional art, and it is an object thereof to provide a catalyst for production of carboxylic acid ester, the catalyst exhibiting high activity while suppressing outflow of the catalyst.

Solution to Problem

The present inventors have completed the present invention by finding that the object can be attained by setting a bulk density and a particle diameter distribution ($D_{10}/D_{50}$, $D_{90}/D_{50}$, and $W/D_{50}$) to predetermined ranges.

That is, the present invention encompasses aspects as follows.

[1] A catalyst for production of carboxylic acid ester, comprising:

catalyst metal particles; and
a support supporting the catalyst metal particles,
wherein a bulk density of the catalyst for production of carboxylic acid ester is 0.50 g/cm$^3$ or more and 1.50 g/cm$^3$ or less,
when a particle diameter, at which a cumulative frequency is x% in a particle diameter distribution based on a volume of the catalyst for production of carboxylic acid ester, is defined as $D_x$, $D_{10}/D_{50} \geq 0.2$ and $D_{90}/D_{50} \leq 2.5$ are satisfied, and
when a half-width of the particle diameter distribution is defined as W, $W/D_{50} \leq 1.5$ is satisfied.

[2] The catalyst for production of carboxylic acid ester according to [1], wherein the W is 100 μm or less.

[3] The catalyst for production of carboxylic acid ester according to [1] or [2], wherein the catalyst metal particles comprise at least one element selected from the group consisting of nickel, cobalt, palladium, platinum, ruthenium, lead, gold, silver, and copper.

[4] The catalyst for production of carboxylic acid ester according to any of [1] to [3], wherein the catalyst metal particles are composite particles comprising:

oxidized nickel and/or cobalt, and
X, wherein X represents at least one element selected from the group consisting of nickel, palladium, platinum, ruthenium, gold, silver, and copper.

[5] The catalyst for production of carboxylic acid ester according to [4], wherein a compositional ratio between nickel or cobalt and X in the composite particles, in terms of a Ni/X atomic ratio or a Co/X atomic ratio, is 0.1 to 10.

[6] The catalyst for production of carboxylic acid ester according to [4] or [5], wherein the composite particles comprise oxidized nickel or cobalt and gold.

[7] The catalyst for production of carboxylic acid ester according to any of [4] to [6], wherein an average particle diameter of the composite particles is 2 to 10 nm.

[8] The catalyst for production of carboxylic acid ester according to any of [4] to [7], wherein a supported layer, in which the composite particles are localized, is present in a region extending from a surface of the catalyst for production of carboxylic acid ester to 40% of an equivalent diameter of the catalyst for production of carboxylic acid ester.

[9] The catalyst for production of carboxylic acid ester according to any of [4] to [8],

wherein the equivalent diameter is 200 μm or less, and
the supported layer, in which the composite particles are localized, is present in a region extending from the surface of the catalyst for production of carboxylic acid ester to 30% of the equivalent diameter of the catalyst for production of carboxylic acid ester.

[10] The catalyst for production of carboxylic acid ester according to any of [4] to [9],

wherein the catalyst for production of carboxylic acid ester has an outer layer substantially free of composite particles on the outside of the supported layer, in which the composite particles are localized, and
the outer layer is formed at a thickness of 0.01 to 15 μm.

[11] The catalyst for production of carboxylic acid ester according to any of [4] to [10], wherein the composite particles have a core composed of X, and the core is coated with oxidized nickel and/or cobalt.

[12] The catalyst for production of carboxylic acid ester according to any of [1] to [11], wherein the support comprises silica and alumina.

[13] The catalyst for production of carboxylic acid ester according to any of [1] to [12], wherein the $D_{50}$ is 10 μm or more and 200 μm or less.

[14] A method for producing carboxylic acid ester comprising a step of reacting (a) an aldehyde and an alcohol or (b) one or two or more alcohols in a presence of the catalyst for production of carboxylic acid ester according to any of [1] to [13] and oxygen.

[15] The method for producing carboxylic acid ester according to [14], wherein the aldehyde is acrolein and/or methacrolein.

[16] The method for producing carboxylic acid ester according to [14] or [15], wherein the aldehyde is acrolein and/or methacrolein, and the alcohol is methanol.

Advantageous Effects of Invention

According to the present invention, a catalyst for production of carboxylic acid ester that exhibits high activity while suppressing outflow of the catalyst can be provided.

Description of Embodiment

Hereinbelow, an embodiment of the present invention (hereinbelow, referred to as "the present embodiment") will be described in detail. The present invention is not limited to the following present embodiment, and can be variously modified and implemented without departing from the gist.

[Catalyst for production of carboxylic acid ester]

A catalyst for production of carboxylic acid ester of the present embodiment comprises catalyst metal particles and a support supporting the catalyst metal particles, wherein a bulk density of the catalyst for production of carboxylic acid ester is 0.5 $g/cm^3$ or more and 1.5 $g/cm^3$ or less, a half-width of a particle diameter distribution based on the volume of the catalyst for production of carboxylic acid ester is 100 $\mu$m or less, when a particle diameter, at which a cumulative frequency is x% in a particle diameter distribution based on the volume of the catalyst for production of carboxylic acid ester, is defined as $D_x$, $D_{10}/D_{50} \geq 0.2$ and $D_{90}/D_{50} \leq 2.5$ are satisfied, and when the half-width of the particle diameter distribution based on the volume is defined as W, $W/D_{50} \leq 1.5$ is satisfied. Being thus configured, the catalyst for production of carboxylic acid ester of the present embodiment exhibits high activity while suppressing outflow of the catalyst.

In the present embodiment, the bulk density of the catalyst for production of carboxylic acid ester is 0.50 $g/cm^3$ or more and 1.50 $g/cm^3$ or less. Although the bulk density may be considered less relevant to a problem in relation to reduction in catalyst activity, it is considered that, since the bulk density adjusted to the above range tends to facilitate diffusing the catalyst in a reactor, the conversion rate of a raw material is increased. That is, it is considered that when the bulk density is smaller than the above lower limit value, the activity is reduced because the amount of the catalyst is decreased due to outflow of the catalyst from a reactor in long-term operation; and when the bulk density is larger than the above upper limit value, the activity is reduced because the chance of contact with a raw material is hindered due to low circularity of the catalyst. From such viewpoints, the bulk density of the catalyst for production of carboxylic acid ester is 0.70 $g/cm^3$ or more and 1.30 $g/cm^3$ or less, and more preferably 0.90 $g/cm^3$ or more and 1.20 $g/cm^3$ or less.

The bulk density can be measured by a method described in the examples to be described below.

The bulk density can be adjusted to the above range, for example, by adopting preferable production conditions described below.

When a particle diameter, at which a cumulative frequency is x% in a particle diameter distribution based on the volume of the catalyst for production of carboxylic acid ester, is defined as $D_x$, $D_{10}/D_{50}$ is 0.2 or more. $D_{10}/D_{50}$ is an index that indicates how many particles having a small particle diameter based on an average particle diameter are present as the catalyst for production of carboxylic acid ester. A numeric value thereof closer to 1.0 means that a sharper distribution on the small particle side. In the present embodiment, when $D_{10}/D_{50}$ is 0.2 or more, the fluidity of the catalyst for production of carboxylic acid ester is enhanced, and high activity is exerted. From similar viewpoints, $D_{10}/D_{50}$ is preferably 0.3 to 0.8.

$D_{10}/D_{50}$ can be measured by a laser diffraction/scattering method and, more specifically, can be measured by a method described in the examples to be described below.

$D_{10}/D_{50}$ can be adjusted to the above range, for example, by adopting preferable production conditions described below.

In the present embodiment, $D_{90}/D_{50}$ is 2.5 or more. This is an index that indicates how many particles having a large particle diameter based on an average particle diameter are present as the catalyst for production of carboxylic acid ester. A numeric value thereof closer to 1.0 means that a sharper distribution on the large particle side. In the present embodiment, when $D_{90}/D_{50}$ is 2.5 or more, the fluidity of the catalyst for production of carboxylic acid ester is enhanced, and high activity is exerted. From similar viewpoints, $D_{90}/D_{50}$ is preferably 1.4 to 2.3.

$D_{90}/D_{50}$ can be measured by a laser diffraction/scattering method and, more specifically, can be measured by a method described in the examples to be described below.

$D_{90}/D_{50}$ can be adjusted to the above range, for example, by adopting preferable production conditions described below.

When a half-width of the particle diameter distribution based on the volume of the catalyst for production of carboxylic acid ester is defined as W, $W/D_{50}$ is 1.5 or less. $W/D_{50}$ is an index that indicates how broad a peak half-width in the particle diameter distribution based on an average particle diameter is. A smaller numeric value thereof means a sharper distribution. In the present embodiment, when $W/D_{50}$ is 1.5 or less, the fluidity of the catalyst for production of carboxylic acid ester is enhanced while the outflow of the catalyst is suppressed, and high activity is exerted while the outflow of the catalyst is suppressed. When the particle diameter distribution has a plurality of peaks, W as to the highest peak satisfies the above relationship. From similar viewpoints, $W/D_{50}$ is preferably 0.6 to 1.3.

$W/D_{50}$ can be measured by a laser diffraction/scattering method and, more specifically, can be measured by a method described in the examples to be described below.

$W/D_{50}$ can be adjusted to the above range, for example, by adopting preferable production conditions described below.

In the present embodiment, the W is preferably 100 $\mu$m or less. When the value of W is 100 $\mu$m or less, the outflow of the catalyst tends to be more suppressed. Although not only the bulk density described above but the half-width may be considered less relevant to a problem in relation to reduction in catalyst activity, the half-width adjusted to the above range prevents the outflow of the catalyst from a reactor and further tends to increase the conversion rate of a raw material accordingly. It has not been known so far and is a novel finding that the particle diameter distribution of the catalyst could result in the outflow of the catalyst from a reactor to thereby influence the conversion rate of a raw material.

From similar viewpoints, W is more preferably 5 $\mu$m or more and 95 $\mu$m or less, and even more preferably 10 $\mu$m or more and 90 $\mu$m or less.

W can be measured by a laser diffraction/scattering method and, more specifically, can be measured by a method described in the examples to be described below.

W can be adjusted to the above range, for example, by adopting preferable production conditions described below.

In the present embodiment, $D_{50}$ is preferably 10 $\mu$m or more and 200 $\mu$m or less, more preferably 20 $\mu$m or more and 150 $\mu$m or less, even more preferably 40 $\mu$m or more and 100 $\mu$m or less, and still even more preferably 40 $\mu$m or more and 80 $\mu$m or less. When $D_{50}$ is in the above range, the resulting catalyst for production of carboxylic acid ester tends to exhibit higher activity while more suppressing outflow of the catalyst.

$D_{50}$ can be measured by a laser diffraction/scattering method and, more specifically, can be measured by a method described in the examples to be described below.

$D_{50}$ can be adjusted to the above range, for example, by adopting preferable production conditions described below.

In the present embodiment, the particle diameter distribution of the catalyst preferably has a single peak. The particle diameter distribution having a single peak means that the particle diameter distribution based on the volume does not have a peak of 1/5 or more of the highest peak, other than the highest peak.

In the present embodiment, the catalyst metal particles are not particularly limited as long as the catalyst metal particles have a function of catalyzing a reaction for producing carboxylic acid ester. From the viewpoint of catalyst performance, it is preferable to comprise at least one element selected from the group consisting of nickel, cobalt, palladium, platinum, ruthenium, lead, gold, silver, and copper. From the viewpoint of further catalyst performance, the catalyst metal particles are more preferably composite particles comprising: oxidized nickel and/or cobalt, and X, wherein X represents at least one element selected from the group consisting of nickel, palladium, platinum, ruthenium, gold, silver, and copper. Further preferably, the composite particles comprise oxidized nickel or cobalt and gold.

In the present embodiment, the catalyst for production of carboxylic acid ester preferably has a supported layer in which the composite particles are localized. The term "supported layer in which the composite particles are localized" refers to a region in the support where composite particles are concentratedly supported. In the catalyst for production of carboxylic acid ester of the present embodiment, composite particles are preferably supported not randomly in the support but selectively in a certain region. This region is referred to as the "supported layer in which the composite particles are localized". In the catalyst for production of carboxylic acid ester, if the composite particles are concentrated in a certain region as compared with other portions, the region is the "supported layer in which the composite particles are localized". Thus, which region is a "supported layer in which the composite particles are localized" can be determined by X-ray microprobe analysis described below or from secondary electron reflected images obtained with a high-resolution scanning electron microscope. The supported layer in which the composite particles are localized is preferably present in a region extending from the surface of the catalyst for production of carboxylic acid ester to 40% of the equivalent diameter of the catalyst for production of carboxylic acid ester. When the supported layer in which the composite particles are localized is present in this region, the influence of the diffusion rate of the reaction material within the support is reduced, and the reaction activity tends to be improved.

The catalyst for production of carboxylic acid ester of the present embodiment can have various sizes including a substantial thickness or particle diameter on the order of $\mu$m to cm, and various shapes. Specific examples of the shape of the catalyst for production of carboxylic acid ester include, but are not limited to, various shapes such as spheres, ovals, cylinders, tablets, hollow cylinders, plates, rods, sheets, and honeycombs. The shape can be suitably changed according to the reaction form, and in a fixed bed reaction, for example, hollow cylindrical or honeycomb-shaped particles are selected due to their low pressure loss, while spherical particles are typically selected under conditions of suspension of a liquid phase slurry.

The term "equivalent diameter" referred to herein represents the diameter of spherical particles, or in the case of irregularly shaped particles, the diameter of a sphere having an equal volume as the particles or having a surface area equal to the surface area of the particles. For a method for measuring the equivalent diameter, $D_{50}$ is measured using a laser diffraction/scattering particle size distribution measuring apparatus, and the resulting value is taken as the equivalent diameter.

The optimum range of the thickness of the supported layer in which the composite particles are localized is selected according to the thickness of the support, particle diameter, type of reaction, and reaction form. Since the "equivalent diameter of the catalyst for production of carboxylic acid ester" is usually the same as the "equivalent diameter of the support", the "equivalent diameter of the catalyst for production of carboxylic acid ester" can be determined from the equivalent diameter of the support.

On the other hand, when the equivalent diameter of the catalyst for production of carboxylic acid ester is 200 $\mu$m or less, the composite particles are preferably supported in the region extending from the surface of the catalyst for production of carboxylic acid ester to 30% of the equivalent diameter of the catalyst for production of carboxylic acid ester. In the case of using in a liquid phase reaction, in particular, the support has been designed to have a small particle diameter in line with the reaction because the influence of the reaction rate and the intrapore diffusion rates of reaction materials within the support occurs. In the present embodiment, reducing the thickness of the supported layer in which the composite particles are localized enables a highly active catalyst for production of carboxylic acid ester to be obtained without reducing the particle diameter of the support. In this case, separation of the catalyst by settling is facilitated, and additionally, the catalyst can be advantageously separated using a small-volume separator. Meanwhile, if the volume of the portion in which composite particles in the catalyst for production of carboxylic acid ester are not supported becomes excessively large, the volume not required by the reaction per reactor increases, thereby resulting in waste in some cases. Accordingly, it is preferable to set the particle diameter of the support according to the reaction form and to set the required thickness of the supported layer in which the composite particles are localized and the thickness of the layer in which composite particles are not supported.

The catalyst for production of carboxylic acid ester may also have an outer layer substantially free of composite particles on the outside of the supported layer in which the composite particles are localized. The outer layer is preferably formed at a thickness of from 0.01 to 15 $\mu$m from the outer surface of the support. Providing the outer layer within this range enables the catalyst to be used as a catalyst that is strongly resistant to catalyst poisoning and from which falling-off of composite particles due to abrasion is suppressed, in reactions using a reactor such as a fluidized bed, bubble column, stirring reactor, or any other reactor for which friction of catalyst particles is of concern, and in reactions in which there occurs accumulation of poisoning substances. In addition, the outer layer can be controlled to be extremely thin, and thus large decrease in activity can be suppressed.

The optimum range of the thickness of the outer layer substantially free of composite particles is selected according to the reaction characteristics, physical properties of the support, amount of composite particles supported, and the like, and is preferably from 0.01 to 15 $\mu$m, more preferably from 0.1 to 10 $\mu$m, and even more preferably from 0.2 to 5 $\mu$m. If the thickness of the outer layer (composite particle-non-supported layer) is more than 15 $\mu$m, the catalyst activity may be reduced, though the effect of improving the life of the catalyst is not changed in use of the composite particles as a catalyst. If the thickness of the outer layer is less than 0.01 $\mu$m, falling-off of composite particles due to abrasion tends to easily occur.

In the present embodiment, the term "substantially free of composite particles" means the substantial absence of a peak indicating the distribution of oxidized nickel and/or cobalt and X (wherein X represents at least one of elements selected from the group consisting of nickel, palladium, platinum, ruthenium, gold, silver, and copper) having a relative intensity of 100 or more in X-ray microprobe analysis described below or in secondary electron reflected images obtained with a high-resolution scanning electron microscope.

Preferable examples of the oxidized nickel capable of constituting the composite particles according to the present embodiment include nickel oxides formed by bonding nickel and oxygen (e.g., $Ni_2O$, $NiO$, $NiO_2$, $Ni_3O_4$, or $Ni_2O_3$), or composite oxides containing nickel such as nickel oxide compounds formed by bonding nickel and X and/or one or more other metal elements and oxygen, or a solid solution or mixture thereof.

Preferable examples of the oxidized cobalt capable of constituting the composite particles according to the present embodiment include cobalt oxides formed by bonding cobalt and oxygen (e.g., $CoO$, $Co_2O_3$, or $Co_3O_4$), or composite oxides containing cobalt such as cobalt oxide compounds formed by bonding cobalt and X and/or one or more other metal elements and oxygen, or a solid solution or mixture thereof.

The term "nickel oxide" referred to herein represents a compound containing nickel and oxygen. Nickel oxides include the previously exemplified $Ni_2O$, $NiO$, $NiO_2$, $Ni_3O_4$, $Ni_2O_3$, or hydrates thereof, hydroperoxides of nickel containing an OOH group, peroxides of nickel containing an $O_2$ group, and mixtures thereof.

The term "composite oxide" referred to herein represents an oxide containing two or more metals. The "composite oxide" refers to an oxide in which two or more metal oxides form a compound. Although including multiple oxides not containing an ion of an oxoacid as a structural unit (e.g., perovskite oxides and spinel oxides of nickel), the composite oxide has a broader concept than that of multiple oxides, including all oxides in which two or more metals are compounded. Oxides in which two or more metal oxides form a solid solution are also within the scope of composite oxides.

In the catalyst for production of carboxylic acid ester of the present embodiment, on compounding nickel oxide and/or cobalt oxide with X as described above, the inherent catalytic ability of nickel oxide and/or cobalt oxide having oxidative esterification activity can be brought out, and a markedly high level of catalyst performance, which cannot be achieved with a catalyst composed of each component singly, tends to appear. This is thought to be because a unique effect developed by compounding nickel oxide and/or cobalt oxide and X, that is, a novel catalyst action completely different from that of each component singly, is generated by a dual functional effect between both the metal components, the formation of a new active species, or the like. Further, in the case of supporting oxidized nickel and/or oxidized cobalt and X on a support in a highly dispersed state, revolutionary catalyst performance unable to be obtained with conventional catalysts tends to be achieved.

For example, if gold is selected for X and nickel oxide and gold are supported on a support in a highly dispersed state, markedly high catalyst performance tends to appear. Such a catalyst for production of carboxylic acid ester has higher selectivity for carboxylic acid ester, in comparison with a catalyst in which each of nickel oxide or gold is supported singly on the support, and tends to exhibit largely improved activity at a specific compositional ratio of Ni/Au. The catalyst also exhibits high catalytic activity per metal atom as compared with a particle supported material composed of each component singly, and development of catalyst performance caused by the compounding is strongly dependent on the composition of nickel and gold supported. This is presumed to be due to the presence of an optimum ratio for formation of a nickel oxidation state that is optimum for the reaction. Since the two components: the nickel oxide and gold are dispersed and supported on a support in this manner, highly outstanding compounding effects, which cannot be predicted from the simple combined addition of each component singly, tends to be developed.

The catalyst for production of carboxylic acid ester in which gold is selected for X has the oxidized nickel and gold supported on a support in a highly dispersed state, and both the components tend to be compounded at the nano-size level. When such a catalyst for production of carboxylic acid ester is observed with a transmission electron microscope/scanning transmission electron microscope (TEM/STEM), typically a structure is observed in which nearly spherical nanoparticles of 2 to 3 nm are uniformly dispersed and supported on a support.

When the catalyst is subjected to an elementary analysis of the nanoparticles by energy dispersive X-ray spectrometry (EDS), typically, the catalyst is observed to be in a form in which nickel and gold coexist in all the particles and the surface of gold nanoparticles is coated with nickel, and also a nickel component is observed to be singly supported on the support in addition to the nanoparticles containing nickel and gold.

Further, subjecting the catalyst to X-ray photoelectron spectroscopy (XPS) and powder X-ray diffraction (powder XRD) enables the state of existence of the metals to be confirmed. Typically, while gold is observed to exist as a crystalline metal, nickel is observed to exist as an amorphous oxide having a valence of 2.

Further, when the catalyst is subjected to ultraviolet-visible spectroscopy (UV-Vis), with which change in the excited state of electrons can be observed, typically, a surface plasmon absorption peak (at about 530 nm) originating from gold nanoparticles observed in gold nanoparticles of a single metal species is observed to disappear due to compounding of nickel oxide and gold. The phenomenon of disappearance of this surface plasmon absorption peak has not been observed in catalysts composed of combinations of gold and metal oxide species other than nickel oxide not observed to have any effect on the reaction (e.g., metal oxides such as chromium oxide, manganese oxide, iron oxide, cobalt oxide, copper oxide, and zinc oxide). The disappearance of this surface plasmon absorption peak is thought to be caused by the result of the formation of a mixed electron state via the contact interface between oxidized nickel and gold, that is, hybridization of two metallic chemical species.

Conversion to highly oxidized nickel oxide can be confirmed via color change of the catalyst and ultraviolet-visible spectroscopy (UV-Vis). As a result of adding gold to nickel oxide, the nickel oxide changes in color from grayish green to brown, and the UV spectrum demonstrates absorbance over nearly the entire visible light region. The shape of the UV spectrum and the color of the catalyst are similar to those of a highly oxidized nickel oxide ($NiO_2$) measured as a reference sample. As described above, the nickel oxide is presumed to be converted to a highly oxidized nickel oxide due to the addition of gold.

From the above results, the structure of the composite particles in the case of selecting gold for X is believed to be in a form in which the gold particles serve as the core and the surface thereof is coated with a highly oxidized nickel oxide, without any gold atoms present on the surface of the composite particles.

The composite particles are preferably supported on the support in a highly dispersed state. The composite particles are more preferably dispersed and supported in the form of microparticles or a thin film, and the average particle diameter thereof is preferably from 2 to 10 nm, more preferably from 2 to 8 nm, and even more preferably from 2 to 6 nm.

When the average particle diameter of the composite particles is within the above ranges, a specific active species structure composed of nickel and/or cobalt and X is formed, and the reaction activity tends to improve. Here, the average particle diameter of the composite particles in the present embodiment means the number average particle diameter as measured with a transmission electron microscope (TEM). Specifically, in an image observed with a transmission electron microscope, an area of black contrast indicates composite particles. The diameter of each of all the particles is measured, and the number average thereof can be calculated therefrom.

The composition between nickel or cobalt and X in the composite particles, in terms of a Ni/X atomic ratio or a Co/X atomic ratio, is preferably in a range of from 0.1 to 10, more preferably from 0.2 to 8.0, and even more preferably from 0.3 to 6.0. When the Ni/X atomic ratio or the Co/X atomic ratio is within the above range, a specific active species structure composed of nickel and/or cobalt and X, and oxidized nickel and/or cobalt optimal for reaction are formed. As a result, the activity and the selectivity tend to be higher than those when the atomic ratio falls outside the above range.

The form of the composite particles is not particularly limited as long as both of the components of nickel and/or cobalt and X are included. Preferable is a form that includes both of the components preferably coexisting in the particles and has a phase structure, that is, any one of structures such as a solid solution structure in which chemical species randomly occupy crystal sites, a core-shell structure in which each chemical species is separated in the shape of concentric spheres, an anisotropic phase separation structure in which phases are separated anisotropically, or a heterobondphilic structure in which both chemical species are present adjacent to each other on the particle surface. More preferable is a form in which the particles have a core composed of X and the surface of the cores is coated with oxidized nickel and/or cobalt. The shape of the composite particles is not particularly limited as long as both the components are contained therein, and may take any shape such as spherical, hemispherical, or the like.

As described above, transmission electron microscopy/scanning transmission electron microscopy (TEM/STEM), for example, is effective as an analysis technique for observing the form of the composite particles. Elementary analyses within the particles and visualization of images of the distribution of elements therein are enabled by irradiating nanoparticles observed by TEM/STEM with an electron beam. The composite particles in the present embodiment contain nickel and/or cobalt and X in all of the particles as will be indicated in the examples to be described below, and have been confirmed to have a form in which the surface of X is coated with nickel and/or cobalt. In the case in which the particles have such a form, the atomic ratio of nickel and/or cobalt to X varies according to the locations of the composition analysis points in the particles, and nickel and/or cobalt is detected in larger amounts on the edges of the particles than in the central portion thereof. Thus, the atomic ratio of nickel or cobalt to X may vary depending on the locations of the analysis points even among individual particles, and the range thereof is included in the range of the Ni/X atomic ratio or the Co/X atomic ratio as described above.

In the case in which gold, silver, or copper is selected for X, ultraviolet-visible spectroscopy (UV-Vis) is an effective means of identifying the structure thereof. In the case of nanoparticles of gold, silver, or copper singly, the photoelectric field in the visible to near infrared region is coupled with free electrons on the surface of the metal, indicating surface plasmon absorption. For example, when a catalyst including gold particles supported is irradiated with visible light, an absorption spectrum is observed that is based on plasmon resonance originating from gold particles at a wavelength of about 530 nm. However, in the catalyst for production of carboxylic acid ester including nickel oxide and gold supported according to the present embodiment, since the surface plasmon absorption thereof disappears, gold can be considered not to be present on the surface of the composite particles in the present embodiment.

The solid form of the nickel is not particularly limited as long as the predetermined activity is obtained, and preferred is an amorphous form having no diffraction peak observed in X-ray diffraction. With such a form, in use as a catalyst for an oxidation reaction, interaction with oxygen is presumed to increase. Additionally, the bonding interface between the oxidized nickel and X increases, and thus better activity tends to be obtained.

In the present embodiment, X is at least one element selected from the group consisting of nickel, palladium, platinum, ruthenium, gold, silver, and copper. More preferably, X is selected from nickel, palladium, ruthenium, gold, and silver.

The chemical state of X may be a metal, an oxide, a hydroxide, a composite compound containing X and nickel, cobalt, or one or more another metal elements, or a mixture thereof, and a preferable chemical state is a metal or oxide and more preferably a metal. The solid form of X is not particularly limited as long as the predetermined activity can be obtained, and the form may be either a crystalline form or an amorphous form.

The term "another metal element" herein refers to a third component element or a metal component such as an alkali metal, an alkaline earth metal, or a rare earth metal to be contained in the catalyst for production of carboxylic acid ester, besides a constituent element of the support to be described below, oxidized nickel and/or cobalt and X.

The catalyst for production of carboxylic acid ester of the present embodiment has oxidized nickel and/or cobalt and X supported on a support as described above, and exerts excellent effects as a result of forming composite particles composed of oxidized nickel and/or cobalt and X. The term "composite particle" herein refers to a particle containing different binary metal species in a single particle. Examples of binary metal species different from this include binary metal particles in which both nickel and/or cobalt and X are metals and metal particles forming an alloy or intermetallic compound of nickel and/or cobalt and X. In the case of using these as catalysts for chemical synthesis, the selectivity of the target product and the catalyst activity tend to be lower as compared with the catalyst for production of carboxylic acid ester of the present embodiment.

The catalyst for production of carboxylic acid ester of the present embodiment preferably contains oxidized nickel and/or cobalt singly on the support in addition to the composite particles composed of oxidized nickel and/or cobalt and X. The presence of oxidized nickel and/or cobalt not compounded with X further enhances the structural stability of the catalyst for production of carboxylic acid ester and inhibits increase in the pore diameter caused by a prolonged reaction as well as the accompanying growth of the composite particles. This effect becomes marked in the case of using an aluminum-containing silica-based composition containing silica and alumina as the support, as will be described below.

Hereinafter, there will be described action that enhances the structural stability of the catalyst for production of carboxylic acid ester and that inhibits increase in the pore diameter caused by a prolonged reaction as well as the accompanying growth of composite particles by allowing oxidized nickel and/or cobalt singly to be present on the support.

As will be described below, in a synthesis reaction of carboxylic acid ester, by-production of acetal or the like caused by acidic substances exemplified by methacrylic acid or acrylic acid, which is a by-product unique to the production reaction of carboxylic acid ester, can be inhibited by adding a compound of an alkali metal or alkaline earth metal to the reaction system to maintain the pH of the reaction system at 6 to 9, and more preferably at neutral conditions (e.g., pH 6.5 to 7.5), that is, as close to pH 7 as possible.

According to studies conducted by the present inventors, in the case of carrying out a prolonged reaction according to the reaction procedure using a gold particle supported material in which single-component gold particles are supported on a support composed of an aluminum-containing silica-based composition containing silica and alumina, structural changes tend to occur, although gradually, in the gold particle supported material. This phenomenon is thought to be caused by increase in the pore diameter of the supported material particles, which increase is brought about because the supported material particles are locally and repeatedly exposed to acid and base by the reaction procedure, and thus a portion of the Al in the support dissolves and precipitates, resulting in rearrangement of the silica-alumina crosslinked structure. In addition, accompanying the change of increase in the pore diameter, sintering of the gold particles occurs to cause the surface area to be decreased, and thus the catalyst activity tends to decrease.

On the other hand, the presence of composite particles and oxidized nickel and/or cobalt singly on the support enhances the structural stability of the supported material particles according to the reaction procedure described above, and increase in the pore diameter and growth of the composite particles tend to be inhibited. As described above, the formation of a nickel and/or cobalt oxide compound or a composite oxide containing nickel and/or cobalt in the form of a solid solution and the like due to reaction of oxidized nickel and/or cobalt with constituent elements of the support is considered to be a factor behind the reason. As a result of action of such a nickel compound on stabilization of the silica-alumina crosslinked structure, structural change in the supported material particles is thought to have greatly improved. The present inventors have presumed that the appearance of the structural stabilizing effect of this supported material is attributable to the oxidized nickel and/or cobalt present in the support. For this reason, this effect is naturally obtained when oxidized nickel and/or cobalt contained in composite particles is in contact with the support, and an even greater stabilizing effect is thought to be obtained when oxidized nickel and/or cobalt is present singly on the support.

The support of the catalyst for production of carboxylic acid ester of the present embodiment is not particularly limited as long as the support can support oxidized nickel and/or cobalt and X, and conventional catalyst supports used for chemical synthesis can be used.

Examples of supports include various types of supports such as activated carbon, silica, alumina, silica-alumina, titania, silica-titania, zirconia, magnesia, silica-magnesia, silica-alumina-magnesia, calcium carbonate, zinc oxide, zeolite, and crystalline metallosilicate. Preferable examples of supports include activated carbon, silica, alumina, silica-alumina, silica-magnesia, silica-alumina-magnesia, titania, silica-titania, and zirconia, and more preferable examples include silica-alumina and silica-alumina-magnesia.

The support may contain one or more metal components selected from alkali metals (Li, Na, K, Rb, and Cs), alkaline earth metals (Be, Mg, Ca, Sr, and Ba), and rare earth metals (La, Ce, and Pr). Metal components to be supported on the support are preferably metal components that become oxides by calcining or the like, such as nitrates or acetates.

A support composed of an aluminum-containing silica-based composition containing silica and aluminum is preferably used for the support. In other words, the support preferably contains silica and alumina. The support has higher water resistance than that of silica and higher acid resistance than that of alumina. The support comprises characteristics superior to those of conventional supports typically used, including greater hardness and higher mechanical strength than those of activated carbon, and is also able to stably support the active components: oxidized nickel and/or cobalt and X. Consequently, the catalyst for production of carboxylic acid ester becomes capable of maintaining high reactivity over a long period.

The catalyst for production of carboxylic acid ester having a specific atomic ratio for oxidized nickel and/or cobalt and X and including an aluminum-containing silica-based composition for the support, when used as a catalyst for chemical synthesis, has high mechanical strength, be physically stable, and satisfies corrosion resistance with respect to liquid properties unique to the reaction while having a large surface area suitable for use as a catalyst support.

Hereinafter, there will be described the characteristics of a support composed of an alumina-containing silica-based composition containing silica and alumina of the present embodiment, which has enabled the catalyst life to be considerably improved. Reasons why the mechanical strength and chemical stability of the support have been able to be greatly improved are presumed as follows.

In the support composed of an aluminum-containing silica-based composition, addition of aluminum (Al) to an uncrosslinked silica (Si-O) chain of a silica gel leads to new formation of Si-O-Al-O-Si bonds, and it is considered that the formation of an Al-crosslinked structure without any loss of the inherent stability to acidic substances of the Si-O chain has allowed the Si-O bonds to be strengthened and the stability of hydrolysis resistance (hereinafter, simply referred to as "water resistance") to be considerably improved. When a Si-O-Al-O-Si crosslinked structure is formed, the number of uncrosslinked Si-O chains decreases in comparison with the case of silica gel singly, and also the mechanical strength is thought to increase. That is, the number of Si-O-Al-O-Si structures formed and improvement of the mechanical strength and water resistance of the resulting silica gel is presumed to be correlated.

One of the reasons why the oxidized nickel and/or cobalt and X can be stably supported on a support for a long period is that the support has greatly improved mechanical strength and chemical stability, as described above, and comprises superior physical properties in comparison with typically used conventional supports. As a result, it is considered that nickel and/or cobalt and X, the active components, are unlikely to be separated from the support and can be stably supported over a long period.

In a commonly used support such as silica or silica-titania, nickel and/or cobalt components have been confirmed to elute from the support, although gradually, in prolonged reactions. In contrast, the present inventors have found that when the support described above is used, elution of nickel and/or cobalt components is suppressed over a long period. On the basis of the results of X-ray photoelectron spectroscopy (XPS), transmission electron microscopy (TEM/EDX), and a double crystal high-resolution X-ray fluorescence method (HRXRF), when a silica or silica-titania support is used, eluted nickel and/or cobalt components have been confirmed to be nickel oxide or cobalt oxide present singly on the support. Since nickel oxide or cobalt oxide is a compound soluble in acid, when used for a catalyst for carboxylic acid ester synthesis, the oxide is presumed to be eluted by an acidic substance typified by methacrylic acid or acrylic acid that are unique by-products of the reaction.

On the basis of analyses of the chemical state of nickel and/or cobalt by a double crystal high-resolution X-ray fluorescence method (HRXRF), as for the nickel and/or cobalt in the catalyst for production of carboxylic acid ester of the present embodiment, it is presumed that there has been formed not only nickel oxide and/or cobalt oxide as a single compound but also a composite oxide containing nickel and/or cobalt such an oxidized compound of nickel and/or cobalt formed as a result of bonding between nickel oxide and/or cobalt oxide and constituent component elements of the support, or a solid solution or mixture thereof.

The double crystal high-resolution X-ray fluorescence method (HRXRF) has extremely high energy resolution and is able to analyze a chemical state from the energy levels (chemical shifts) and shapes of the resulting spectrum. In the Kα spectra of 3d transition metal elements in particular, changes appear in the chemical shift and shape due to changes in the valence or electronic state, thereby making it possible analyze the chemical state in detail. In the catalyst for production of carboxylic acid ester of the present embodiment, changes appear in the NiKα spectrum, and a chemical state has been confirmed for nickel that differs from that of nickel oxide as a single compound.

For example, nickel aluminate, which is formed from nickel oxide and alumina, is a compound insoluble in acid. As a result of forming such a nickel compound on a support, elution of the nickel component is presumed to have been greatly improved.

As for the elementary composition of the support composed of an aluminum-containing silica-based composition containing silica and alumina, the amount of aluminum is in the range of 1 to 30 mol%, preferably 5 to 30 mol%, and more preferably 5 to 25 mol%, based on the total molar amount of silicon and the aluminum. When the amount of aluminum is in the above range, acid resistance and mechanical strength tend to be satisfactory.

It is preferable that the support in the catalyst for production of carboxylic acid ester of the present embodiment further comprise an oxide of at least one basic metal selected from alkali metals, alkaline earth metals, and rare earth metals, in addition to silica and alumina, from the viewpoint of more improving mechanical strength and chemical stability. Examples of alkali metals of the basic metal components include Li, Na, K, Rb, and Cs, examples of alkaline earth metals include Be, Mg, Ca, Sr and Ba, and examples of rare earth metals include La, Ce, and Pr. Among those described above, Mg as an alkaline earth metal is preferable, and it is especially preferable that the support comprise silica, alumina, and magnesia.

As for the elementary composition of the support comprising silica, alumina, and an oxide of at least one basic metal selected from alkali metals, alkaline earth metals, and rare earth metals, the amount of aluminum is in the range of 1 to 30 mol%, preferably 5 to 30 mol%, and more preferably 5 to 25 mol%, based on the total molar amount of silicon and the aluminum. The compositional ratio between the basic metal oxide and alumina, in terms of (alkali metal + 1/2 × alkaline earth metal + 1/3 × rare earth metal) / Al atomic ratio, is in the range of preferably 0.5 to 10, more preferably 0.5 to 5.0, and even more preferably 0.5 to 2.0. When the elementary composition of silica, alumina, and the basic metal oxide is in the above range, silicon, aluminum, and the basic metal oxide form a specific stable bonding structure, and the resulting support tends to have satisfactory mechanical strength and water resistance.

Next, a preferable method for preparing the support will be described by taking the case of using an aluminum-containing silica-based composition containing silica and alumina as an example, though the method for preparing the support is not limited thereto. That is, the aluminum-containing silica-based composition is prepared by reacting a silica sol with an aluminum compound solution, and the support is prepared therefrom.

In the above example, a silica sol is used for the silica source. In this case, after obtaining a mixed sol containing a silica sol and an aluminum compound by mixing the silica sol and the aluminum compound, the mixed sol is subjected to a multi-stage hydrothermal reaction at 20 to 100°C for 1 to 48 hours and dried to obtain a gel followed by calcining under temperature, time and atmospheric conditions described below. Alternatively, it is preferable to add an alkaline aqueous solution to the above mixed sol followed by coprecipitating the silica and an aluminum compound, carrying out drying and then calcining under conditions described below. It is also possible to obtain a support composed of an aluminum-containing silica-based composition having a desired particle diameter by a step such as a step of micronizing the above mixed sol directly using a spray dryer, a step of drying the mixed sol followed by granulating the gel, or the like.

In a method for preparing a support containing silica, alumina, and an oxide of at least one of basic metals including alkali metals, alkaline earth metals, and rare earth metals, in accordance with the above method for preparing a support composed of an aluminum-containing silica-based composition containing silica and alumina, the support can be prepared by drying a slurry obtained by mixing an alkali metal oxide, alkaline earth metal oxide and/or rare earth metal oxide into silica and aluminum components, and further calcining the dried slurry under conditions described below.

A common commercially available compound can be used as a raw material of the alkali metal, alkaline earth metal, or rare earth metal in the same manner as the aluminum raw material. The raw material is preferably a water-soluble compound and more preferably a hydroxide, carbonate, nitrate, or acetate.

Another preparation method that can be used is a method in which a basic metal component selected from the group consisting of alkali metals, alkaline earth metals, and rare earth metals is adsorbed onto a support composed of an aluminum-containing silica-based composition. For example, there can be applied a method using an immersion method in which a support is added to a liquid including a basic metal compound dissolved followed by drying treatment or the like, or a method using an impregnation method in which a basic compound equivalent to the pore volume is allowed to permeate into a support followed by drying treatment. However, a method in which the basic metal component is adsorbed later requires caution such as conducting liquid drying treatment under mild conditions for allowing the basic metal component to be highly dispersed in the support.

An inorganic substance or organic substance can be added to the mixed slurry of the various raw materials described above, in order to control slurry properties and finely adjust the pore structure or other characteristics of the product or the properties of the resulting support.

Specific examples of inorganic substances to be used include mineral acids such as nitric acid, hydrochloric acid, and sulfuric acid, salts of metals including alkali metals such as Li, Na, K, Rb, and Cs and alkaline earth metals such as Mg, Ca, Sr, and Ba, water-soluble compounds such as ammonia or ammonium nitrate, and clay minerals that are dispersed in water to form a suspension. Specific examples of organic substances include polymers such as polyethylene glycol, methyl cellulose, polyvinyl alcohol, polyacrylic acid, and polyacrylamide.

There are various effects of addition of inorganic substances and organic substances, and the main effects include formation of a spherical support, controlling the pore diameter and pore volume, and the like. Specifically, the liquid properties of the mixed slurry are an important factor in obtaining a spherical support. Adjusting the viscosity and solid content using an inorganic substance or inorganic substance enables the liquid properties to be altered so as to easily obtain a spherical support. The control of the pore diameter and pore volume can be carried out by a multi-stage hydrothermal synthesis step of the mixed slurry described below. It is also preferable to appropriately select and use an optimum organic compound that remains inside the support at the molding stage of the support and the residual substance of which can be removed by calcining and washing operations after the molding.

(Method for producing catalyst for production of carboxylic acid ester)

The method for producing a catalyst for production of carboxylic acid ester is not particularly limited. According to a method described below, the catalyst for production of carboxylic acid ester having a bulk density in a predetermined range and a predetermined particle diameter distribution according to the present embodiment is easily obtained.

A preferable method for producing a catalyst for production of carboxylic acid ester according to the present embodiment comprises: step I of spray-drying a mixed slurry in a dryer of rotating disk type; step II of calcining the obtained spray-dried product to obtain a support; and step III of allowing the support to support catalyst metal particles. In the method for producing a catalyst for production of carboxylic acid ester according to the present embodiment, the amount of the mixed slurry fed by spraying based on the radius in the lateral direction of the spray dryer in the step I is $5 \times 10^{-3}$ m$^2$/Hr or more and $70 \times 10^{-3}$ m$^2$/Hr or less, and the circumferential speed of the disk is 10 m/s or more and 120 m/s or less.

According to the above method, a bulk density in a predetermined range and a predetermined particle diameter distribution in the catalyst for production of carboxylic acid ester according to the present embodiment are easily obtained, and the resulting catalyst for production of carboxylic acid ester exhibits high activity while suppressing outflow of the catalyst.

In preparing the mixed slurry for use in the step I according to the present embodiment, for example, a stirring operation of a raw material mixed solution can be carried out while heating conditions are appropriately adjusted.

The support according to the present embodiment can be produced by spray-drying a mixed slurry containing various raw materials described above and an additive, as in the step I and the step II. A known spraying apparatus such as a rotating disk type, two-fluid nozzle type, or pressurized nozzle type can be used, for a method for converting the mixed slurry into droplets.

The liquid to be sprayed (mixed slurry) is preferably used in a well-mixed state (well-dispersed state of each component). Such a satisfactory mixed state tends to reduce a detrimental effect on the performance of the support, such as decreased durability caused by uneven distribution of each component. Particularly, on formulating the raw material mixed solution, increases in the slurry viscosity and partial gelling (colloidal condensation) may occur, resulting in a concern of formation of non-uniform particles. For this reason, it may be preferable that formulation of the raw material mixed solution is performed by methods including, for example, adding acid or alkali to control the mixed slurry to around pH 2, namely, a semi-stable state for the silica sol, besides giving attention to gradually mix the raw materials while stirring.

The liquid to be sprayed (mixed slurry) preferably has certain degrees of a viscosity and a solid content. When the viscosity and the solid content are above certain levels, a porous material obtained by spray drying tends to be prevented from forming indentations without forming a perfect sphere. When the viscosity and the solid content are below certain levels, the dispersibility of porous materials can be secured. In addition, this tends to contribute to stable formation of droplets. Change in viscosity for 1 hour from 1 hour before the start of spray drying (raw material mixed solution state) to spraying (mixed slurry state) (final $\Delta$ viscosity) tends to affect a particle diameter distribution and a bulk density obtained by spray drying.

From the above viewpoints, the final $\Delta$ viscosity is preferably less than 10 mPa·s/Hr, more preferably 7 mPa·s/Hr or less, and even more preferably 5 mPa·s/Hr or less. The lower limit value of the final $\Delta$ viscosity is not particularly limited and is, for example, 0 mPa·s/Hr or more. The final $\Delta$ viscosity can be measured on the basis of a method described in the examples to be described below.

The solid content is preferably in the range of 10 to 50% by mass in view of a shape, a bulk density, and a particle diameter.

The tip speed of a stirring blade in stirring the raw material mixed solution is not particularly limited and is preferably 3 m/s or more and 9 m/s or less, more preferably 4 m/s or more and 8 m/s or less, and even more preferably 5 m/s or more and 7 m/s or less, from the viewpoint of control of the final $\Delta$ viscosity.

In the case of spray-drying a mixed slurry in a dryer of rotating disk type, the following conditions are preferable. The dryer of rotating disk type has a disk of, for example, an atomizer. By using the disk, the bulk density and the particle size distribution of a dried product can be adjusted by spraying the mixed slurry.

Specifically, the amount of the liquid fed by spraying based on the radius in the lateral direction of the spray dryer is $5 \times 10^{-3}$ m$^2$/Hr or more and $70 \times 10^{-3}$ m$^2$/Hr or less, more preferably $10 \times 10^{-3}$ m$^2$/Hr or more and $50 \times 10^{-3}$ m$^2$/Hr or less, and even more preferably $20 \times 10^{-3}$ m$^2$/Hr or more and $40 \times 10^{-3}$ m$^2$/Hr or less.

The circumferential speed of the disk is 10 m/s or more and 120 m/s or less, more preferably 20 m/s or more and 100 m/s or less, and even more preferably 30 m/s or more and 90 m/s or less.

The support calcining temperature is typically selected within a range of from 200 to 800°C. Calcining at a temperature above 800°C is not preferable because the specific surface area tends to decrease considerably. The calcining atmosphere is not particularly limited, and calcining is typically carried out in air or nitrogen. The calcining time can be determined according to the specific surface area after calcining, and is generally from 1 to 48 hours. For calcining conditions, the porosity and other properties of the support may change, and thus selection of suitable temperature conditions and heating conditions is required. If the calcining temperature is excessively low, it tends to be difficult to maintain durability for a composite oxide, while if the calcining temperature is excessively high, there is the risk of causing decrease in the pore volume. The temperature rise conditions are preferably such that the temperature is raised gradually using programmed temperature rising or the like. A case of abrupt calcining under high temperature conditions is not preferable because gasification and combustion of inorganic substances and organic substances becomes violent, the support is exposed to a high-temperature state beyond the setting, and this exposure may cause the support to be pulverized.

The particle diameter distribution can also be controlled by adding a classification step after calcining or after catalyst supporting. Examples of the classification apparatus include sieves, shaking sieving machines, inertial classifiers, forced vortex centrifugal classifiers, and free vortex-type classifiers such as cyclones.

From the viewpoints of ease of supporting composite particles, reaction activity in the case of using as a catalyst, difficulty in separation, and reaction activity, the specific surface area of the support is preferably 10 $m^2$/g or more, more preferably 20 $m^2$/g or more, and even more preferably 50 $m^2$/g or more as measured by BET nitrogen adsorption. There is no particular limitations from the viewpoint of activity, but from the viewpoints of mechanical strength and water resistance, the specific surface area is preferably 700 $m^2$/g or less, more preferably 350 $m^2$/g or less, and even more preferably 300 $m^2$/g or less.

If the pore diameter is smaller than 3 nm, the separation properties of the supported metal tend to be satisfactory, but in the case of using as a catalyst in a liquid phase reaction or the like, the pore diameter is preferably 3 nm or more from the viewpoints of not making the intrapore diffusion resistance excessively great so as not to cause the diffusion process of the reaction substrate to be rate-limiting as well as of maintaining the reaction activity at a high level. On the other hand, the pore diameter is preferably 50 nm or less from the viewpoints of difficulty in cracking of the supported material and difficulty in separation of the supported metal. Thus, the pore diameter of the support is preferably from 3 to 50 nm and more preferably from 3 to 30 nm. The pore volume is required for the presence of pores supporting composite nanoparticles. However, if the pore volume increases, the strength tends to suddenly decrease. Accordingly, from the viewpoints of strength and supporting characteristics, the pore volume is preferably within a range of from 0.1 to 1.0 mL/g and more preferably within a range of from 0.1 to 0.5 mL/g. The support of the present embodiment preferably satisfies the above ranges for both the pore diameter and pore volume.

The shape of the support depends on the reaction form. For a fixed bed reaction, a hollow cylindrical or honeycomb form, which results in little pressure loss, is selected. Under conditions of a liquid phase slurry suspension, generally chosen is a spherical form for which an optimum particle diameter is selected for use in accordance with the reactivity and the separation method. For example, in the case of employing a generally simple catalyst separation process based on precipitation separation, a particle diameter to be selected is preferably from 10 to 200 $\mu$m, more preferably from 20 to 150 $\mu$m, and even more preferably from 30 to 150 $\mu$m, in consideration of the balance with reaction characteristics. In the case of a cross filter system, small particles of 0.1 to 20 $\mu$m or less are preferable because of its higher reactivity. The type and form of the support can be changed according to the purpose of use for use in a catalyst for chemical synthesis.

The amount of oxidized nickel or cobalt supported on the support is not particularly limited, and is usually from 0.01 to 20% by mass, preferably from 0.1 to 10% by mass, more preferably from 0.2 to 5% by mass, and even more preferably from 0.5 to 2% by mass, as nickel or cobalt, based on the mass of the support. The amount of X supported on the support, in terms of metal, is usually from 0.01 to 10% by mass, preferably from 0.1 to 5% by mass, more preferably from 0.2 to 2% by mass, even more preferably from 0.3 to 1.5% by mass, and particularly preferably from 0.5 to 1.0% by mass, based on the mass of the support.

Moreover, in the present embodiment, a preferable range exists for the atomic ratio between nickel and/or cobalt and the above-mentioned constituent elements of the support. In the case of using the support composed of an aluminum-containing silica-based composition containing silica and alumina in the present embodiment, the compositional ratio between nickel or cobalt and alumina in a catalyst, in terms of the Ni/Al atomic ratio or the Co/Al atomic ratio, is preferably from 0.01 to 1.0, more preferably from 0.02 to 0.8, and even more preferably from 0.04 to 0.6. In the case of using a support containing silica, alumina, and an oxide of at least one basic metal of alkali metals, alkaline earth metals, and rare earth metals, the compositional ratio between nickel or cobalt and alumina in the supported material, in terms of the atomic ratio of Ni/Al or the Co/Al atomic ratio, is preferably from 0.01 to 1.0, more preferably from 0.02 to 0.8, and even more preferably from 0.04 to 0.6, and the compositional ratio between nickel or cobalt and the basic metal component, in terms of the Ni/(alkali metal + alkaline earth metal + rare earth metal) atomic ratio or the Co/(alkali metal + alkaline earth metal + rare earth metal) atomic ratio, is preferably from 0.01 to 1.2, more preferably from 0.02 to 1.0, and even more preferably from 0.04 to 0.6.

When the atomic ratios of nickel and/or cobalt to aluminum and basic metal oxide as the support constituent elements are within the above ranges, the effects of improving elution of nickel and/or cobalt and structural changes in supported material particles tend to increase. This is thought to be because the nickel and/or cobalt, aluminum, and basic metal oxide form a specific composite oxide within the above ranges, thereby forming a stable bonding structure.

The catalyst for production of carboxylic acid ester of the present embodiment can contain a third constituent element, as an activity component, in addition to oxidized nickel and/or cobalt and X. Examples of third constituent elements that can be contained include titanium, vanadium, chromium, manganese, iron, zinc, gallium, zirconium, niobium, molybdenum, rhodium, cadmium, indium, tin, antimony, tellurium, hafnium, tantalum, tungsten, rhenium, osmium, iridium, mercury, thallium, lead, bismuth, aluminum, boron, silicon, and phosphorous. The content of these third constituent elements in the supported material is preferably from 0.01 to 20% by mass and more preferably from 0.05 to 10% by mass. At least one metal component selected from alkali metals, alkaline earth metals, and rare earth metals may also be contained in the catalyst for production of carboxylic acid ester. The content of the alkali metal, alkaline earth metal, or rare earth metal is preferably selected to be within a range of 15% by mass or less in the supported material.

These third constituent elements or alkali metal, alkaline earth metal and rare earth metal may be contained in the supported material during production or reaction of the catalyst for production of carboxylic acid ester, or a method may be used in which these are contained in the support in advance.

The specific surface area of the catalyst for production of carboxylic acid ester of the present embodiment is preferably within a range of from 20 to 350 m$^2$/g, more preferably from 50 to 300 m$^2$/g, and even more preferably from 100 to 250 m$^2$/g, as measured by BET nitrogen adsorption, from the viewpoints of reaction activity and resistance to separation of active components.

The pore diameter of the catalyst for production of carboxylic acid ester is originating from the pore structure of the support. If the pore diameter is smaller than 3 nm, the separation properties of the supported metal component tend to be satisfactory. However, in the case of using as a catalyst in a liquid phase reaction or the like, the pore diameter is preferably 3 nm or more, from the viewpoints of not making the intrapore diffusion resistance excessively large so as not to cause the diffusion process of the reaction substrate to be rate-limiting as well as of maintaining the reaction activity at a high level. On the other hand, the pore diameter is preferably 50 nm or less from the viewpoints of difficulty in cracking of the supported material and difficulty in separation of the supported composite particles. Thus, the pore diameter of the catalyst for production of carboxylic acid ester is preferably from 3 to 50 nm, more preferably from 3 to 30 nm, and even more preferably from 3 to 10 nm. From the viewpoints of supporting characteristics and reaction characteristics, the pore volume is preferably within a range of from 0.1 to 1.0 mL/g, more preferably from 0.1 to 0.5 mL/g, and even more preferably from 0.1 to 0.3 mL/g. The catalyst for production of carboxylic acid ester of the present embodiment preferably satisfies the above ranges for both the pore diameter and pore volume.

[Method for producing catalyst for production of carboxylic acid ester]

The method for producing a catalyst for production of carboxylic acid ester of the present embodiment is not particularly limited and can include the following preferable steps. Hereinafter, each step will be described.

In a first step, an aqueous slurry containing a support supporting an oxide of at least one basic metal selected from the group consisting of alkali metals, alkaline earth metals, and rare earth metals is mixed with an acidic aqueous solution of soluble metal salts containing nickel and/or cobalt and X, wherein X represents at least one element selected from the group consisting of nickel, palladium, platinum, ruthenium, gold, silver, and copper. The temperature of the mixture of both liquids is adjusted so as to be 60°C or more. A precursor of a catalyst for production of carboxylic acid ester in which the nickel and/or cobalt and the component X have precipitated on the support is formed in the mixture.

Next, in a second step, the precursor obtained in the first step is washed with water and dried as necessary followed by subjecting to heat treatment, and thus the catalyst for production of carboxylic acid ester can be obtained.

According to this method, a catalyst for production of carboxylic acid ester can be obtained that has a supported layer in which the composite particles are localized but does not contain composite particles in a region that includes the center of the support.

In the present embodiment, prior to the first step, it is preferable to carry out an aging step of aging the support supporting an oxide of at least one basic metal selected from the group consisting of alkali metals, alkaline earth metals, and rare earth metals in water. Aging the support in advance allows a layer having a sharp distribution of composite particles to be obtained. The effect caused by aging the support is presumed, based on the results of measuring pore distribution by nitrogen adsorption, to be attributable to a more uniform and sharper pore structure due to occurrence of realignment of the pore structure of the support. Although the aging can be carried out at room temperature, the aging temperature for the support is preferably selected within a range of from 60 to 150°C, which is higher than room temperature, because the pore structure changes slowly. In the case in which the water dispersion is carried out at normal pressure, a temperature within a range of from 60 to 100°C is preferable. The duration of the aging varies according to the temperature conditions. In the case of 90°C, for example, the duration is preferably from 1 minute to 5 hours, more preferably from 1 to 60 minutes, and even more preferably from 1 to 30 minutes. In the operation of the first step, although the support can be used after aged, then once dried, and calcined, a slurry obtained by dispersing the support in water is preferably brought into contact with the acidic aqueous solution of soluble metal salts containing nickel and/or cobalt and X followed by insoluble immobilization of nickel and/or cobalt and the component X on the support.

Examples of soluble metal salts containing nickel for use in the preparation of the catalyst include nickel nitrate, nickel acetate, and nickel chloride. Examples of soluble metal salts containing X include palladium chloride and palladium acetate in the case of selecting palladium for X, ruthenium chloride and ruthenium nitrate in the case of selecting ruthenium for X, chloroauric acid, sodium tetrachloroaurate, potassium dicyanoaurate, gold diethylamine trichloride, and gold cyanide in the case of selecting gold for X, and silver chloride and silver nitrate in the case of selecting silver for X.

The concentration of each of the aqueous solutions containing nickel and/or cobalt and X are usually within a range of from 0.0001 to 1.0 mol/L, preferably from 0.001 to 0.5 mol/L, and more preferably from 0.005 to 0.2 mol/L. The ratio of nickel or cobalt to X in the aqueous solutions, in terms of the Ni/X atomic ratio or the Co/X atomic ratio, is preferably within a range of from 0.1 to 10, more preferably from 0.2 to 5.0, and even more preferably from 0.5 to 3.0.

The temperature during contact between the support and the acidic aqueous solution of soluble metal salts containing nickel and/or cobalt and X is one important factor for controlling the distribution of composite particles, and varies according to the amount of the oxide of at least one basic metal selected from the group consisting of alkali metals, alkaline earth metals, and rare earth metals supported in advance on the support. If the temperature is excessively low, the reaction slows, and the distribution of the composite particles tends to widen. In the production method of the present embodiment, from the viewpoint of obtaining a supported layer in which the composite particles are more sharply localized, the temperature during contact with the acidic aqueous solution of soluble metal salts containing nickel and/or cobalt and X is a temperature at which a high reaction rate is achieved, and is preferably 60°C or more, more preferably 70°C or more, even more preferably 80°C or more, and particularly preferably 90°C or more. Since the acidic aqueous solution and the aqueous slurry may be mixed so that the temperature of the mixed liquid thereof is 60°C or more, the aqueous slurry may be heated to a degree such that the mixed liquid exceeds 60°C even after the acidic aqueous solution is added, or conversely only the acidic aqueous solution may be heated. It is natural that both the acidic aqueous solution and the aqueous slurry may also be heated to 60°C or more.

The reaction can be carried out under applied pressure at a temperature equal to or higher than the boiling point of the solution, and usually preferably carried out at a temperature equal to or lower than the boiling point for the ease of the operation. The duration of immobilization of the nickel and/or cobalt and X components is not particularly limited, and depends on conditions such as the type of support, the amount of nickel and/or cobalt and X supported and the ratio thereof. The duration is usually within a range of from 1 minute to 5 hours, preferably from 5 minutes to 3 hours, and more preferably from 5 minutes to 1 hour.

The method for producing a catalyst for production of carboxylic acid ester of the present embodiment is based on, for example, the principle of insoluble immobilization of the nickel and/or cobalt and X component by carrying out a chemical reaction between an oxide of at least one basic metal selected from the group consisting of alkali metals, alkaline earth metals, and rare earth metals supported in advance on the support and soluble metal salts containing nickel and/or cobalt and X. In order to ensure more sufficient compounding of the nickel and/or cobalt and X component, both the components are preferably immobilized simultaneously from a mixed solution containing both the components.

In the production method of the present embodiment, the aqueous slurry containing a support supporting an oxide of at least one basic metal selected from the group consisting of alkali metals, alkaline earth metals, and rare earth metals preferably contains a salt of at least one basic metal selected from the group consisting of alkali metals, alkaline earth metals, and rare earth metals.

Thus, formation of metal black of X can be inhibited, compounding of nickel and/or cobalt and X is facilitated, and the distribution of composite particles can be controlled more precisely. Such effects are presumed to be caused by controlling the rate of the chemical reaction between the basic metal oxide supported in advance on the support and the soluble metal salts containing nickel and/or cobalt and X by means of addition of a salt of at least one metal selected from the group consisting of alkali metals, alkaline earth metals and rare earth metals to the aqueous solution.

Examples of salts of at least one basic metal selected from the group consisting of alkali metals, alkaline earth metals, and rare earth metals that can be used include one or more selected from water-soluble salts of these metals such as organic acid salts, and inorganic salts including nitrates and chlorides.

The amount of the salt of at least one basic metal selected from the group consisting of alkali metals, alkaline earth metals, and rare earth metals described above varies according to the amounts and ratio of the nickel and/or cobalt and X component, and is determined according to the amount of the basic metal oxide supported in advance on the support. Usually, the amount of the salt is from 0.001 to 2 times moles and preferably from 0.005 to 1 time moles, based on the amount of the nickel and/or cobalt and X component in the aqueous solution.

The aqueous slurry containing a support supporting an oxide of at least one basic metal selected from the group consisting of alkali metals, alkaline earth metals, and rare earth metals preferably contains a soluble aluminum salt. Examples of soluble aluminum salts that can be used include aluminum chloride and aluminum nitrate.

Addition of a soluble aluminum salt to the aqueous slurry can lead to formation of an outer layer substantially free of composite particles on the outside of the supported layer in which the composite particles are localized. This is also based on the principle of insoluble immobilization as described above. A soluble salt such as aluminum chloride or aluminum nitrate is used for the soluble aluminum salt. Aluminum is allowed to react on the outer surface of the support by a chemical reaction with the basic metal oxide supported in advance on the support, the reaction field for nickel and/or cobalt and X is consumed, and the basic metal oxide further inside and the nickel and/or cobalt and X are immobilized by a reaction.

The amount of the aluminum component varies according to the setting of the thickness ($\mu$m) of the layer not supporting the nickel and/or cobalt and X component thereon, and is determined according to the amount of the basic metal oxide supported in advance on the support. Usually, the amount of the aluminum component is from 0.001 to 2 times moles and preferably from 0.005 to 1 time mole, based on the amount of the basic metal oxide supported in advance on the support.

Although much is still unknown about the details of the mechanism by which the nickel and/or cobalt and X component are distributed, it is presumed that the diffusion rate of the nickel and/or cobalt and X-containing soluble component in the support and the rate at which the components are insolubilized by a chemical reaction were well-balanced under the conditions of the present embodiment, thereby enabling the composite particles to be immobilized in a narrow region near the surface of the support.

In the case of forming an outer layer substantially free of composite particles on the outer surface of the support, aluminum and a basic metal component near the outer surface of the support are allowed to react with each other to lead to consumption of the basic metal component that may react with the nickel and/or cobalt and X component near the outer surface of the support, and when nickel and/or cobalt and X are subsequently supported, the reactive basic metal component near the outer surface of the support is already consumed. Accordingly, the nickel and/or cobalt and X are presumed to react with the basic metal oxide inside the support followed by being immobilized.

Next, the second step will be described.

The first precursor is washed with water and dried as necessary prior to the heat treatment of the second step. The heating temperature for the first precursor is usually from 40 to 900°C, preferably from 80 to 800°C, more preferably from 200 to 700°C, and even more preferably from 300 to 600°C.

The heat treatment is carried out in an atmosphere such as air (or atmospheric air), an oxidizing atmosphere (such as oxygen, ozone, nitrogen oxides, carbon dioxide, hydrogen peroxide, hypochlorous acid, or inorganic or organic peroxide), or an inert gas atmosphere (such as helium, argon, or nitrogen). The heating time is only required to be suitably selected according to the heating temperature and the amount of the first precursor. The heat treatment can be carried out at normal pressure, under applied pressure, or under reduced pressure.

Following the second step described above, a reduction treatment can be carried out in a reducing atmosphere (such as hydrogen, hydrazine, formalin, or formic acid) as necessary. In this case, a treatment method selected and carried out in which the oxidized nickel and/or cobalt are not completely reduced to a metallic state. The temperature and duration of the reducing treatment are suitably selected according to the type of the reducing agent, the type of X, and the amount of the catalyst.

Further, following the above-mentioned heat treatment or reducing treatment, oxidizing treatment can be carried out in air (or atmospheric air) or an oxidizing atmosphere (such as oxygen, ozone, nitrogen oxide, carbon dioxide, hydrogen peroxide, hypochlorous acid, or inorganic or organic peroxide), as necessary. The temperature and duration in this case is suitably selected according to the type of the oxidizing agent, the type of X, and the amount of the catalyst.

A third constituent element other than nickel and/or cobalt and X can be added during preparation of the supported material or under reaction conditions. The alkali metal, alkaline earth metal, or rare earth metal can also be added during catalyst preparation or to the reaction system. The raw materials for the third constituent element, alkali metal, alkaline earth metal, and rare earth metal are selected from salts of organic acids, salts of inorganic acids, hydroxides, and the like.

[Method for producing carboxylic acid ester]

The catalyst for production of carboxylic acid ester of the present embodiment can be widely used as a catalyst for chemical synthesis. For example, the catalyst can be used in reactions for forming carboxylic acid esters from aldehydes and alcohols and reactions for forming carboxylic acid esters from alcohols. In other words, the method for producing carboxylic acid ester of the present embodiment can include a step of reacting (a) an aldehyde and an alcohol or of (b) one or two or more alcohols in the presence of the catalyst for production of carboxylic acid ester of the present embodiment and oxygen.

The catalyst for production of carboxylic acid ester of the present embodiment exerts excellent effects particularly when used as a catalyst for oxidization reactions. In addition to the aldehydes and alcohols used in the reaction for forming carboxylic acid ester as indicated in the examples, examples of reaction substrates used in the present embodiment include various reaction substrates such as alkanes, olefins, alcohols, ketones, aldehydes, ethers, aromatic compounds, phenols, sulfur compounds, phosphorous compounds, oxygen-containing nitrogen compounds, amines, carbon monoxide, and water. These reaction substrates can be used singly or as a mixture composed of two or more thereof. Various industrially useful oxidation products such as oxygen-containing compounds, oxidative adducts, and oxidative dehydrogenation products are obtained from these reaction substrates.

As specific reaction substrates, examples of alkanes include aliphatic alkanes such as methane, ethane, propane, n-butane, isobutane, n-pentane, n-hexane, 2-methylpentane, and 3-methylpentane, and alicyclic alkanes such as cyclopentane, cyclohexane, cycloheptane, and cyclooctane.

Examples of olefins include aliphatic olefins such as ethylene, propylene, butene, pentene, hexene, heptene, octene, decene, 3-methyl-1-butene, 2,3-dimethyl-1-butene, and allyl chloride; alicyclic olefins such as cyclopentene, cyclohexene, cycloheptene, cyclooctene, and cyclodecene; and aromatic substituted olefins such as styrene and α-methylstyrene.

16

Examples of alcohols include saturated and unsaturated aliphatic alcohols such as methanol, ethanol, n-propanol, isopropanol, n-butanol, s-butanol, t-butanol, n-pentanol, n-hexanol, n-heptanol, allyl alcohol, and crotyl alcohol; saturated and unsaturated alicyclic alcohols such as cyclopentanol, cyclohexanol, cycloheptanol, methylcyclohexanol, and cyclohexen-1-ol; aliphatic and alicyclic polyhydric alcohols such as ethylene glycol, propylene glycol, trimethylene glycol, 1,3-butanediol, 1,2-cyclohexanediol, and 1,4-cyclohexanediol; and aromatic alcohols such as benzyl alcohol, salicyl alcohol, and benzhydrol.

Examples of aldehydes include aliphatic saturated aldehydes such as formaldehyde, acetoaldehyde, propionaldehyde, isobutylaldehyde, and glyoxal; aliphatic $\alpha,\beta$-unsaturated aldehydes such as acrolein, methacrolein, and crotonaldehyde; aromatic aldehydes such as benzaldehyde, tolylaldehyde, benzylaldehyde, and phthalaldehyde; and derivatives of these aldehydes.

Examples of ketones include aliphatic ketones such as acetone, methyl ethyl ketone, diethyl ketone, dipropyl ketone, and methyl propyl ketone; alicyclic ketones such as cyclopentanone, cyclohexanone, cyclooctanone, 2-methylcyclohexanone, and 2-ethylcyclohexanone; and aromatic ketones such as acetophenone, propiophenone, and benzophenone.

Examples of aromatic compounds include benzene, toluene, xylene, naphthalene, anthracene, or derivatives thereof obtained by substitution with an alkyl group, an aryl group, a halogen, a sulfone group, or the like.

Examples of phenols include phenol, cresol, xylenol, naphthol, anthrol (hydroxyanthracene), and derivatives thereof (such as those obtained by replacing a hydrogen atom in the aromatic ring with an alkyl group, an aryl group, a halogen atom, a sulfonic acid group, or the like).

Examples of sulfur compounds include mercaptans such as methyl mercaptan, ethyl mercaptan, propyl mercaptan, benzyl mercaptan, and thiophenol.

Examples of amines include aliphatic amines such as methylamine, ethylamine, propylamine, isopropylamine, butylamine, dimethylamine, diethylamine, dipropylamine, diisopropylamine, dibutylamine, trimethylamine, triethylamine, tripropylamine, tributylamine, allylamine, and diallylamine; alicyclic amines such as cyclopentylamine, cyclohexylamine, cycloheptylamine, and cyclooctylamine; and aromatic amines such as aniline, benzylamine, and toluidine.

These reaction substrates can be used singly or as a mixture composed of two or more thereof. The reaction substrates are not necessarily required to be purified, and may be in a form of mixture with other organic compounds.

Hereinafter, a method for producing carboxylic acid ester from an aldehyde and an alcohol in the presence of oxygen by an oxidative esterification reaction using the catalyst for production of carboxylic acid ester of the present embodiment will be described by way of example.

Examples of the aldehyde used as a raw material include $C_1$ to $C_{10}$ aliphatic saturated aldehydes such as formaldehyde, acetoaldehyde, propionaldehyde, isobutylaldehyde, and glyoxal; $C_3$ to $C_{10}$ alicyclic $\alpha\cdot\beta$-unsaturated aldehydes such as acrolein, methacrolein, and crotonaldehyde; $C_6$ to $C_{20}$ aromatic aldehydes such as benzaldehyde, tolylaldehyde, benzylaldehyde, and phthalaldehyde; and derivatives of these aldehydes. These aldehydes can be used singly or as a mixture of any two or more thereof. In the present embodiment, the aldehyde is preferably selected from acrolein, methacrolein, or a mixture of these.

Examples of alcohols include $C_1$ to $C_{10}$ aliphatic saturated alcohols such as methanol, ethanol, isopropanol, butanol, 2-ethylhexanol, and octanol; $C_5$ to $C_{10}$ alicyclic alcohols such as cyclopentanol and cyclohexanol; $C_2$ to $C_{10}$ diols such as ethylene glycol, propylene glycol, and butanediol; $C_3$ to $C_{10}$ aliphatic unsaturated alcohols such as allyl alcohol and methallyl alcohol; $C_6$ to $C_{20}$ aromatic alcohols such as benzyl alcohol; and hydroxyoxetanes such as 3-alkyl-3-hydroxymethyloxetane. These alcohols can be used singly or as a mixture of any two or more thereof. In the present embodiment, it is preferable that the aldehyde be acrolein and/or methacrolein and the alcohol be methanol.

The amount ratio of the aldehyde to the alcohol is not particularly limited, and the reaction can be carried out over a wide range of a molar ratio of aldehyde/alcohol of from 10 to 1/1,000. The reaction is generally carried out within the range of a molar ratio of from 1/2 to 1/50.

The amount of catalyst used can be varied considerably according to the types of reaction raw materials, the composition of the catalyst, preparation method, reaction conditions and reaction form, and the like and is not particularly limited. In the case of allowing the catalyst to react in a slurry form, the amount of catalyst used, in terms of the solid content in the slurry, is preferably within the range of from 1 to 50 mass/vol%, more preferably from 3 to 30 mass/vol%, and even more preferably from 10 to 25 mass/vol%.

The production of carboxylic acid ester may be carried out as a batch or continuous process by any method such as a vapor phase reaction, liquid phase reaction, or irrigation liquid reaction.

The reaction can be carried out in the absence of solvent, and can also be carried out using a solvent that is inert to the reaction components such as hexane, decane, benzene, dioxane, or the like.

The reaction can be carried out by means of a conventionally known form, such as a fixed bed form, a fluid bed form, or a stirred tank form. For example, when carried out in the liquid phase, the reaction can be carried out by means of any form of reaction vessel, such as a bubble column reactor, a draft tube reactor, or a stirred tank reactor.

The oxygen used to produce carboxylic acid ester can be molecular oxygen, that is, can be in the form of oxygen gas itself or a mixed gas in which oxygen gas has been diluted with a diluent inert to the reaction such as nitrogen or carbon dioxide gas. Air is preferably used for the oxygen raw material from the viewpoints of ease of manipulation, economy, and the like.

The oxygen partial pressure varies according to the reaction raw materials such as the type of aldehyde or type of alcohol, the reaction conditions or the type of reactor, and the like. The oxygen partial pressure at the reactor outlet is practically within the range equal to or lower than the lower limit of the explosive range, and is preferably controlled to, for example, from 20 to 80 kPa. The reaction can be carried out at a reaction pressure within a wide pressure range from reduced pressure to applied pressure, and is usually carried out at a pressure within the range of from 0.05 to 2 MPa. From the viewpoint of safety, the total pressure is preferably set such that the oxygen concentration of the reactor outflow gas does not exceed the explosive limit (e.g., an oxygen concentration of 8%).

In the case of carrying out a reaction for producing carboxylic acid ester in a liquid phase or the like, it is preferable to keep the pH of the reaction system at 6 to 9 by adding an alkali metal or alkaline earth metal compound (e.g., an oxide, a hydroxide, a carbonate, or a carboxylate) to the reaction system. These alkali metal or alkaline earth metal compounds can be used singly or in combination of two or more thereof.

The reaction can be carried out even at a high temperature of 200°C or more, and the reaction temperature during production of carboxylic acid ester is preferably from 30 to 200°C, more preferably from 40 to 150°C, and even more preferably from 60 to 120°C. The reaction time is not particularly limited, cannot be determined unconditionally because of varying according to the set conditions, and is usually from 1 to 20 hours.

Examples

The present embodiment will be now further described with reference to the following examples, but the present embodiment is not limited to the examples by any means.

In examples and comparative examples described below, measurement of various physical properties was carried out by the following methods.

[Viscosity measurement of mixed slurry]

The viscosity was measured at room temperature every hour from the start of stirring of a mixed slurry under the following conditions.

Viscometer: Brook Digital Viscometer "LVDV1M"
Spindle: LV-1
Rotational speed: 100 rpm
Temperature: Room temperature (25°C)

Difference between the viscosity immediately before the start of feed and the viscosity 1 hour before the start of feed in a spray dryer was taken as a final $\Delta$ viscosity.

[Determination of amounts of Ni and X supported and Ni/X atomic ratio]

The concentrations of nickel and X in the composite particle were quantified using model IRIS Intrepid II XDL ICP emission analyzer (ICP-AES, MS) manufactured by Thermo Fisher Scientific K.K.

For preparation of samples, the supported material was weighed into a Teflon decomposition vessel, and nitric acid and hydrogen fluoride were added thereto.

The resulting solution was heated and decomposed in ETHOS Model TC microwave decomposer manufactured by Milestone General followed by evaporating to dryness over a heater. Then, nitric acid and hydrochloric acid were added to the precipitated residue, and the mixture was decomposed under applied pressure in the microwave decomposer. Pure water was added to the resulting decomposition liquid to make a predetermined volume, and this solution was used as the sample.

In a quantification method, quantification was carried out by an internal standard method in the ICP-AES. The contents of nickel and X in the catalyst were determined by subtracting a simultaneously determined operation blank value, and the amount supported and the atomic ratio were calculated.

[Analysis of crystal structure of composite particles]

Analysis was conducted using model Rint 2500 powder X-ray diffraction apparatus (XRD) manufactured by Rigaku Corp. under conditions of a Cu tube for the X-ray source (40 kV, 200 mA), a measuring range of from 5 to 65 deg (0.02 deg/step), a measuring speed of 0.2 deg/min, and slit widths (scattering, divergence, and reception) of 1 deg, 1 deg, and 0.15 mm.

An approach of uniformly spraying onto a nonreflective sample plate and fixing with neoprene rubber was adopted for samples.

[Analysis of chemical state of composite particle metal components]

Analysis was conducted using the model ESCALAB 250 X-ray photoelectron spectroscopy apparatus (XPS) manufactured by Thermo Electron Co., Ltd. under conditions of Al K$\alpha$ at 15 kV $\times$ 10 mA for the excitation source, an analyzed surface area of about 1 mm (shape: oval), and a 0 to 1,100 eV survey scan and an Ni2p narrow scan for the uptake region.

For measurement samples, the composite particle supported material was crushed in an agate mortar, and collected onto a special-purpose powder sample stage followed by XPS measurement.

[Analysis of chemical state of nickel]

Ni K$\alpha$ spectra were measured with model XFRA190 double crystal high-resolution X-ray fluorescence analyzer (HRXRF) manufactured by Technos, and various parameters thus obtained were compared with those of standard substances (nickel metal and nickel oxide) to predict the chemical state such as the valence of nickel in the supported material.

Measurement samples were directly subjected to measurement. The K$\alpha$ spectrum of Ni was measured in the partial spectral mode. In this respect, Ge (220) was used as analyzing crystals. The slit used had a vertical divergence angle of 1°. The excitation voltage and current were set to 35 kV and 80 mA, respectively. Filter paper was then used as an absorber in the case of reference samples, and a counting time was selected for each sample in the case of supported material samples. Measurement was carried out so that the peak intensity of the K$\alpha$ spectra was 3,000 cps or less and 10,000 counts or more. Measurement was repeated five times for each sample, and metal samples were measured before and after each repeated measurement. After smoothing treatment (S-G method, 7 points, 5 times) of the measured spectra, the peak locations, half-width values (FWHM), and asymmetric indices (AI) were calculated, and the peak locations were treated as a chemical shift ($\Delta E$) from the measured value of the metal sample measured before and after measurement of each sample.

[Morphological observation and elementary analysis of composite particles]

TEM bright field images, STEM dark field images, and STEM-EDS compositional analyses (point analysis, mapping, and line analysis) were measured using model 3100FEF transmission electron microscope/scanning transmission electron microscope (TEM/STEM) manufactured by JEOL Ltd.

[acceleration voltage: 300 kV, with energy dispersive X-ray detector (EDX)].

The data analysis software used was Digital Micrograph(TM) Ver. 1.70.16 from Gatan, Inc. for TEM image and STEM image analyses (length measurement and Fourier transform analysis), and NORAN System SIX Ver. 2.0 from Thermo Fisher Scientific K.K. for EDS data analyses (mapping image processing and compositional quantification and calculation).

For measurement samples, the composite particle supported material was crushed in a mortar and then dispersed in ethanol. After performing ultrasonic cleaning for about 1 minute, the powder was dropped onto a Mo microgrid and air-dried to obtain TEM/STEM observation samples.

[Measurement of ultraviolet-visible spectra of composite particles]

Measurement was carried out using model V-550 ultraviolet-visible spectrophotometer (UV-Vis) manufactured by JASCO Corp. [with integrating sphere unit and powder sample holder] at a measuring range of from 800 to 200 nm and a scanning speed of 400 nm/min.

For measurement samples, the composite particle supported material was crushed in an agate mortar and placed in the powder sample holder followed by UV-Vis measurement.

[Shape observation of support and catalyst for production of carboxylic acid ester]

The support and the catalyst for production of carboxylic acid ester were observed using an X-650 scanning electron microscope (SEM) manufactured by Hitachi, Ltd.

[Measurement of $D_{10}$, $D_{50}$, $D_{90}$, and half-width W of catalyst for production of carboxylic acid ester]

0.2 g of the catalyst was collected into a beaker, and 16 mL of purified water was added thereto followed by dispersion for 1 minute using model VCX130 ultrasonic disperse manufactured by Sonic & Materials. Inc. to prepare a sample for measurement. This sample for measurement was subjected to measurement of particle diameter $D_x$ ($D_{10}$, $D_{50}$, and $D_{90}$) at which a cumulative frequency was x% in a particle diameter distribution based on the volume of the catalyst for production of carboxylic acid ester, and half-width W of the particle diameter distribution (volume-based) using model LS230 laser diffraction/scattering particle size distribution measuring apparatus manufactured by Beckman Coulter Inc.

[Specific surface area]

The specific surface area was measured by the BET method using Quadrasorb from Quantachrome Corp.

[Measurement of bulk density (CBD) of catalyst for production of carboxylic acid ester]

As pretreatment, about 120 g of the catalyst for production of carboxylic acid ester was collected into a stainless crucible and dried for 6 hours in a muffle furnace of 150°C. After calcining, the resultant was placed in a desiccator (containing a silica gel) and cooled to room temperature. Subsequently, 100.0 g of the pretreated catalyst for production of carboxylic acid ester was collected and transferred to a 250 mL measuring cylinder, and the measuring cylinder was tap-packed for 15 minutes in a shaker. Thereafter, the surface of the sample in the measuring cylinder was flattened, and the packing volume was read. The bulk density was set to a value obtained by dividing the mass of the catalyst for production of carboxylic acid ester by the packing volume.

[Outflow rate]

The outflow rate was calculated according to the following expression on the basis of the whole amount of the catalyst used in production of carboxylic acid ester described below and the amount of the catalyst remaining after carrying out the production of carboxylic acid ester for 500 hours.

$$\text{Outflow rate} = \{1 - (\text{Amount of the catalyst remaining} / \text{Amount of the catalyst used in the reaction})\} \times 100 \ [\%]$$

Here, the amount of the catalyst remaining was set to the catalyst mass of the catalyst for production of carboxylic acid ester dried at 130°C for 10 hours after carrying out the production of carboxylic acid ester for 500 hours.
That is, a catalyst having a high outflow rate easily outflows from a reactor and is therefore less likely to remain in the reactor. Thus, the amount of the catalyst to be subjected to a reaction is decreased in long-term operation, and the conversion rate tends to be deteriorated.

[Example 1]

(Production of catalyst for production of carboxylic acid ester)

An aqueous solution obtained by dissolving 3.75 kg of aluminum nitrate nonahydrate, 2.56 kg of magnesium nitrate, and 540 g of a 60% by mass nitric acid in 5.0 L of pure water was gradually added dropwise into 20.0 kg of a solution of a silica sol having a colloidal particle diameter of from 10 to 20 nm ($SiO_2$ content: 30% by mass) in a stirred state maintained at 15°C to adjust pH to 1.8. The thus-obtained raw material mixed solution (solid content: 25% by mass) containing the silica sol, aluminum nitrate, and magnesium nitrate was heated to 55°C. Thereafter, the raw material mixed solution was stirred at a tip speed of a stirring blade of 5.5 m/s for 30 hours to obtain a mixed slurry having a final $\Delta$ viscosity of 0.5 mPa·s/Hr.
Thereafter, spray drying was performed under a condition of a disk (atomizer) circumferential speed of 80 m/s in a spray dryer apparatus while the slurry was fed such that the amount of feed / SD radius was $25 \times 10^{-3}$ [$m^2$] to obtain

a solid. The SD radius means the radius in the lateral direction of the spray dryer of rotating disk type used in the present Examples.

Then, the resulting solid was filled to a thickness of about 1 cm into a stainless steel container having an open top, heated in an electric furnace from room temperature to 300°C over 2 hours, and thereafter held for 3 hours. Further, after heated to 600°C over 2 hours and held for 3 hours, the solid was cooled to obtain a support. The resulting support contained silicon, aluminum, and magnesium at 83.3 mol%, 8.3 mol%, and 8.3 mol%, respectively, based on the total molar amount of the silicon, the aluminum, and the magnesium. The form of the support was determined to be nearly spherical based on observations using a scanning electron microscope (SEM).

300 g of the support obtained as described above was dispersed in 1.0 L of water heated to 90°C, and stirred at 90°C for 15 minutes. Next, an aqueous solution containing 16.35 g of nickel nitrate hexahydrate and 12 mL of a 1.3 mol/L aqueous chloroauric acid solution was prepared and heated to 90°C, and the resulting solution was added to the support slurry and further stirred at 90°C for 30 minutes to thereby insolubly immobilize the nickel and gold component onto the support.

Then, after removing the supernatant by allowing to stand and washing several times with distilled water, the washed resultant was filtered. This filtered residue was dried with a drier at 105°C for 10 hours and calcined at 450°C in air for 5 hours in a muffle furnace to obtain a catalyst for production of carboxylic acid ester supporting 1.05% by mass nickel and 0.91% by mass gold (composite particle supported material of $NiOAu/SiO_2-Al_2O_3-MgO$). The Ni/Au atomic ratio of the obtained catalyst for production of carboxylic acid ester was 4.0.

Samples obtained by embedding the resulting composite particle supported material in a resin and polishing were subjected to ray analysis of particle cross sections using an X-ray microprobe (EPMA). It was consequently confirmed that: a region from the outermost surface of the support to a depth of 0.5 $\mu$m had an outer layer substantially free of nickel and gold; nickel and gold were supported in a region from the surface to a depth of 10 $\mu$m; and composite particles were absent inside the support.

Next, as a result of observing the form of the composite particle supported material under a transmission electron microscope (TEM/STEM), it was confirmed that spherical nanoparticles having a local maximum distribution at a particle diameter of from 2 to 3 nm (number average particle diameter: 3.0 nm) were supported by the support. When the nanoparticles were further observed by magnification, lattice fringes corresponding to Au (111) plane spacings were observed in the nanoparticles. As a result of conducting compositional point analysis on individual nanoparticles by STEM-EDS, nickel and gold were detected in all the particles. The average nickel/gold atomic ratio (the number of particles for calculation: 50) of the nanoparticles was 1.05. As a result of further conducting nanoregion analysis of the observed particles, the Ni/Au atomic ratio was 0.90 for the central portions of the particles and 2.56 for the edges of the particles. Only nickel was detected in a very small amount in portions other than the particles. As a result of carrying out similar measurement at 50 points, nickel was detected in a large amount at or near the edges of all the particles. From EDS element mapping, close agreement between the distributions of nickel and gold was observed. From line profiling of the composition, nickel was distributed slightly larger than the distribution of gold in all the directions of scanning.

From the results of powder X-ray diffraction (XRD), no diffraction pattern derived from nickel was observed, confirming that nickel was present in an amorphous state. On the other hand, there existed a broad peak, albeit not being a clear peak, corresponding to crystals of gold. The average crystallite diameter thereof was calculated according to the Scherrer equation and was consequently on the order of 3 nm, which was a value close to the detection limit (2 nm) of the powder X-ray diffraction. As for the chemical state of nickel, the nickel was confirmed to be divalent on the basis of the results of X-ray photoelectron spectroscopy (XPS).

From the results of the double crystal high-resolution X-ray fluorescence method (HRXRF), it was presumed that the chemical state of nickel was a high-spin state of divalent nickel, and difference in the NiK$\alpha$ spectrum revealed that the chemical state differed from that of nickel oxide as a single compound. The catalyst had a NiK$\alpha$ spectrum half-width (FWHM) of 3.470 and a chemical shift ($\Delta$E) of 0.335 on the basis of the measured spectra. The nickel oxide measured as a reference substance had a NiK$\alpha$ spectrum half-width (FWHM) of 3.249 and a chemical shift ($\Delta$E) of 0.344.

As a result of examining the excited state of electrons in this composite particle supported material by ultraviolet-visible spectroscopy (UV-Vis), no surface plasmon absorption peak derived from gold nanoparticles around 530 nm appeared, and broad absorption caused by $NiO_2$ was confirmed in a wavelength region of from 200 to 800 nm.

From the above results, it is presumed that the fine structure of the composite particles has a form in which the surface of the gold nanoparticles is coated with oxidized nickel.

(Physical property evaluation of catalyst for production of carboxylic acid ester)

The specific surface area of the obtained catalyst for production of carboxylic acid ester was determined and was consequently 141 $m^2/g$. As a result of obtaining a particle diameter distribution from results about the catalyst for production of carboxylic acid ester by laser scattering particle size distribution measurement, the particle diameter distribution half-width W was 50 $\mu$m, and the values of $D_{50}$, $D_{10}/D_{50}$, $D_{90}/D_{50}$, and $W/D_{50}$ were 60 $\mu$m, 0.7, 1.5, and 0.8, respectively. The particle diameter distribution had a single peak. The bulk density was measured and was consequently 1.05 g/cm$^3$.

(Production of carboxylic acid ester)

240 g of the resulting catalyst for production of carboxylic acid ester was placed into a stirred stainless steel reactor having a catalyst separator and a liquid phase portion of 1.2 liters followed by carrying out an oxidative carboxylic acid ester formation reaction from an aldehyde and an alcohol while stirring the contents at a tip speed of the stirrer blade of 4 m/s. That is, a solution of 36.7% by mass methacrolein in methanol at 0.6 liters/hr and a solution of 1 to 4% by mass NaOH in methanol at 0.06 liters/hr were continuously supplied to the reactor, air was blown in at a reaction temperature of 80°C and a reaction pressure of 0.5 MPa such that the outlet oxygen concentration was 8.0 vol%, and the concentration of NaOH supplied to the reactor was controlled such that a pH of the reaction system was 7. The reaction product was continuously extracted from the reactor outlet by overflow, and the reactivity was investigated by analyzing by gas chromatography.

At 500 hours after the start of the reaction, the methacrolein conversion rate was 72%, and the selectivity of methyl methacrylate was 95%. The outflow rate of the catalyst was 2%.

[Examples 2 to 7 and Comparative Examples 1 to 7]

Each catalyst for production of carboxylic acid ester was produced in the same manner as in Example 1 except that the mixed slurry preparation conditions and the spray drying conditions in the production of the support were changed as shown in Table 1. Namely, (i) the solid content was adjusted to the value of Table 1 by increasing or decreasing the amount of pure water dissolving aluminum nitrate nonahydrate and magnesium nitrate in the raw material mixed solution, and (ii) the pH was adjusted to the value of Table 1 by increasing or decreasing the amount of a 60% by mass nitric acid. The physical properties of the catalyst for production of carboxylic acid ester were evaluated in the same manner as in Example 1. Further, carboxylic acid ester was produced in the same manner as in Example 1 using this catalyst, and reaction results and the outflow rate of the catalyst were calculated. The particle diameter distributions based on the volumes of the catalysts of Examples 2 to 7 had a single peak. These results are shown in Table 1.

[Table 1]

| | Support production conditions | | | | | | | | Catalyst for production of carboxylic acid ester | | | | | | | Production of carboxylic acid ester |
| | Mixed slurry preparation conditions | | | | | | Spray drying conditions | | | | | | | | | |
| | Solid content % by mass | pH | Temperature °C | Time Hr | Tip speed of stirring blade m/s | Final Δ viscosity mPa·s/Hr | Amount of feed / SD radius x$10^{-3}$ | Atomizer circumferential speed m/s | Half-width (μm) | Bulk density (g/cm³) | Average particle diameter: $D_{50}$ (μm) | $D_{10}/D_{50}$ | $D_{90}/D_{50}$ | $W/D_{50}$ | Outflow rate | Conversion rate / selectivity |
|---|---|---|---|---|---|---|---|---|---|---|---|---|---|---|---|---|
| Example 1 | 25 | 1.8 | 55 | 30 | 5.5 | 0.5 | 25 | 80 | 50 | 1.05 | 60 | 0.7 | 1.5 | 0.8 | 2% | 72% / 95% |
| Example 2 | 30 | 2 | 60 | 30 | 5.5 | 0.5 | 25 | 80 | 48 | 1.07 | 62 | 0.6 | 1.4 | 0.8 | 3% | 70% / 92% |
| Example 3 | 35 | 2.2 | 55 | 35 | 5.1 | 0.5 | 10 | 34 | 60 | 1.09 | 73 | 0.7 | 1.6 | 0.8 | 4% | 68% / 93% |
| Example 4 | 25 | 1.8 | 50 | 40 | 6.4 | 2 | 25 | 80 | 50 | 0.67 | 60 | 0.7 | 1.5 | 0.8 | 7% | 64% / 91% |
| Example 5 | 40 | 1.2 | 60 | 30 | 4.3 | 6 | 25 | 80 | 50 | 1.33 | 60 | 0.7 | 1.5 | 0.8 | 3% | 67% / 93% |
| Example 6 | 20 | 2.0 | 50 | 40 | 7.1 | 4 | 25 | 100 | 65 | 0.90 | 52 | 0.3 | 1.6 | 1.3 | 6% | 65% / 91% |
| Example 7 | 25 | 2.0 | 60 | 28 | 4.0 | 7 | 40 | 90 | 120 | 1.12 | 83 | 0.7 | 2.3 | 1.4 | 3% | 69% / 92% |
| Comparative Example 1 | 25 | 1.8 | 50 | 24 | 3.7 | 10 | 20 | 150 | 70 | 0.82 | 45 | 0.1 | 1.8 | 1.6 | 12% | 56% / 85% |
| Comparative Example 2 | 25 | 1.8 | 50 | 24 | 3.7 | 10 | 20 | 75 | 95 | 0.80 | 60 | 0.8 | 2.6 | 1.6 | 4% | 61% / 87% |
| Comparative Example 3 | 25 | 2 | 15 | 36 | 9.2 | 17 | 20 | 75 | 92 | 0.48 | 62 | 0.4 | 2.0 | 1.5 | 14% | 54% / 80% |
| Comparative Example 4 | 25 | 2 | 15 | 36 | 9.2 | 18 | 20 | 135 | 72 | 0.62 | 41 | 0.1 | 1.9 | 1.8 | 13% | 55% / 85% |
| Comparative Example 5 | 25 | 2 | 15 | 36 | 9.2 | 17 | 5 | 75 | 57 | 0.64 | 45 | 0.1 | 1.5 | 1.3 | 12% | 52% / 82% |
| Comparative Example 6 | 35 | 2 | 20 | 25 | 2.2 | 12 | 20 | 75 | 100 | 1.55 | 56 | 0.6 | 2.8 | 1.8 | 6% | 56% / 82% |
| Comparative Example 7 | 25 | 0.5 | 55 | 20 | 5.5 | 20 | 20 | 140 | 83 | 0.62 | 48 | 0.1 | 1.9 | 1.7 | 11% | 56% / 85% |

**Claims**

1. A catalyst for production of carboxylic acid ester, comprising:

   catalyst metal particles; and
   a support supporting the catalyst metal particles,
   wherein a bulk density of the catalyst for production of carboxylic acid ester is 0.50 g/cm$^3$ or more and 1.50 g/cm$^3$ or less,
   when a particle diameter, at which a cumulative frequency is x% in a particle diameter distribution based on a volume of the catalyst for production of carboxylic acid ester, is defined as $D_x$, $D_{10}/D_{50} \geq 0.2$ and $D_{90}/D_{50} \leq 2.5$ are satisfied, and
   when a half-width of the particle diameter distribution is defined as W, $W/D_{50} \leq 1.5$ is satisfied.

2. The catalyst for production of carboxylic acid ester according to claim 1, wherein the W is 100 $\mu$m or less.

3. The catalyst for production of carboxylic acid ester according to claim 1 or 2, wherein the catalyst metal particles comprise at least one element selected from the group consisting of nickel, cobalt, palladium, platinum, ruthenium, lead, gold, silver, and copper.

4. The catalyst for production of carboxylic acid ester according to any one of claims 1 to 3, wherein the catalyst metal particles are composite particles comprising:

   oxidized nickel and/or cobalt, and
   X, wherein X represents at least one element selected from the group consisting of nickel, palladium, platinum, ruthenium, gold, silver, and copper.

5. The catalyst for production of carboxylic acid ester according to claim 4, wherein a compositional ratio between nickel or cobalt and X in the composite particles, in terms of a Ni/X atomic ratio or a Co/X atomic ratio, is 0.1 to 10.

6. The catalyst for production of carboxylic acid ester according to claim 4 or 5, wherein the composite particles comprise oxidized nickel or cobalt and gold.

7. The catalyst for production of carboxylic acid ester according to any one of claims 4 to 6, wherein an average particle diameter of the composite particles is 2 to 10 nm.

8. The catalyst for production of carboxylic acid ester according to any one of claims 4 to 7, wherein a supported layer, in which the composite particles are localized, is present in a region extending from a surface of the catalyst for production of carboxylic acid ester to 40% of an equivalent diameter of the catalyst for production of carboxylic acid ester.

9. The catalyst for production of carboxylic acid ester according to any one of claims 4 to 8,

   wherein the equivalent diameter is 200 $\mu$m or less, and
   the supported layer, in which the composite particles are localized, is present in a region extending from the surface of the catalyst for production of carboxylic acid ester to 30% of the equivalent diameter of the catalyst for production of carboxylic acid ester.

10. The catalyst for production of carboxylic acid ester according to any one of claims 4 to 9,

    wherein the catalyst for production of carboxylic acid ester has an outer layer substantially free of composite particles on an outside of the supported layer, in which the composite particles are localized, and
    the outer layer is formed at a thickness of 0.01 to 15 $\mu$m.

11. The catalyst for production of carboxylic acid ester according to any one of claims 4 to 10, wherein the composite particles have a core composed of X, and the core is coated with oxidized nickel or cobalt.

12. The catalyst for production of carboxylic acid ester according to any one of claims 1 to 11, wherein the support comprises silica and alumina.

**13.** The catalyst for production of carboxylic acid ester according to any one of claims 1 to 12, wherein the $D_{50}$ is 10 $\mu$m or more and 200 $\mu$m or less.

**14.** A method for producing carboxylic acid ester comprising a step of reacting (a) an aldehyde and an alcohol or (b) one or two or more alcohols, in a presence of the catalyst for production of carboxylic acid ester according to any one of claims 1 to 13 and oxygen.

**15.** The method for producing carboxylic acid ester according to claim 14, wherein the aldehyde is acrolein and/or methacrolein.

**16.** The method for producing carboxylic acid ester according to claim 14 or 15, wherein the aldehyde is acrolein and/or methacrolein, and the alcohol is methanol.

## INTERNATIONAL SEARCH REPORT

| International application No. |
| --- |
| PCT/JP2021/013068 |

A. CLASSIFICATION OF SUBJECT MATTER
B01J 23/89(2006.01)i; C07C 67/39(2006.01)i; C07C 69/54(2006.01)i
FI: B01J23/89 Z; C07C67/39; C07C69/54 Z

According to International Patent Classification (IPC) or to both national classification and IPC

B. FIELDS SEARCHED

Minimum documentation searched (classification system followed by classification symbols)
B01J23/89; C07C67/39; C07C69/54

Documentation searched other than minimum documentation to the extent that such documents are included in the fields searched

| | |
| --- | --- |
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus (JDreamIII); JST7580 (JDreamIII); JSTChina (JDreamIII)

C. DOCUMENTS CONSIDERED TO BE RELEVANT

| Category* | Citation of document, with indication, where appropriate, of the relevant passages | Relevant to claim No. |
| --- | --- | --- |
| A | JP 2008-538323 A (UHDE GMBH) 23 October 2008 (2008-10-23) entire text | 1-16 |
| A | CN 107519892 A (DALIAN INSTITUTE OF CHEMICAL PHYSICS, CHINESE ACADEMY OF SCIENCES) 29 December 2017 (2017-12-29) entire text | 1-16 |
| A | JP 2000-176285 A (STANDARD OIL CO) 27 June 2000 (2000-06-27) entire text | 1-16 |
| A | JP 2011-529493 A (CELANESE INTERNATIONAL CORPORATION) 08 December 2011 (2011-12-08) entire text | 1-16 |
| A | WO 2012/035637 A1 (ASAHI KASEI CHEMICALS CORPORATION) 22 March 2012 (2012-03-22) entire text | 1-16 |

☐ Further documents are listed in the continuation of Box C.　　☒ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 17 May 2021 (17.05.2021) | 25 May 2021 (25.05.2021) |

| Name and mailing address of the ISA/ | Authorized officer |
| --- | --- |
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**
Information on patent family members

International application No.

PCT/JP2021/013068

| Patent Documents referred in the Report | Publication Date | Patent Family | Publication Date |
|---|---|---|---|
| JP 2008-538323 A | 23 Oct. 2008 | US 2008/0286176 A1 entire text WO 2006/111340 A2 EP 1874464 A1 | |
| CN 107519892 A | 29 Dec. 2017 | (Family: none) | |
| JP 2000-176285 A | 27 Jun. 2000 | US 2002/0062039 A1 entire text EP 1008385 A2 KR 10-2000-0047965 A CN 1264621 A | |
| JP 2011-529493 A | 08 Dec. 2011 | US 2010/0029980 A1 entire text WO 2010/014145 A2 EP 2318353 A1 KR 10-2011-0046515 A CN 102149668 A | |
| WO 2012/035637 A1 | 22 Mar. 2012 | US 2013/0172599 A1 entire text EP 2617679 A1 CN 103097296 A KR 10-2013-0058037 A | |

Form PCT/ISA/210 (patent family annex) (January 2015)

**REFERENCES CITED IN THE DESCRIPTION**

**Patent documents cited in the description**

- JP 4803767 B

- JP 4420991 B